# EUROPEAN PATENT APPLICATION

(11) **EP 4 180 421 A1**
(43) Date of publication of application: **17.05.2023**
(21) Application number: 21306592.3
(22) Date of filing: 16.11.2021
(51) Int. Cl.: C07D 209/58, B01J 31/00, C07F 15/00

(54) **IMINIUM SALTS WITH A BARRALENE RING, CORRESPONDING RUTHENIUM COMPLEXES, AND USES THEREOF**

(71) Applicant: Ecole Nationale Supérieure de Chimie de Rennes, 35000 Rennes (FR); Institut National Des Sciences Appliquees, 35708 Rennes (FR); Université de Rennes 1, 35065 Rennes Cedex (FR); University Of California, San Diego, La Jolla, CA 92093-0910 (US); Centre national de la recherche scientifique, 75016 Paris (FR)
(72) Inventor: MAUDUIT, Marc, 35500 VITRE (FR); MORVAN, Jennifer, 56930 PLUMÉLIAU (FR); TALCIK, Jakub, 35700 RENNES (FR); JAZZAR, Rodolphe, LA JOLLA, 92037 (US); BERTRAND, Guy, SOLANA BEACH, 92075 (US); MELAIMI, Mohand-Ameziane, SAN DIEGO, 92130 (US); SERRATO, Melinda Rachel, LA JOLLA, 92037 (US)
(74) Representative: Lavoix

(57) **Abstract**

The present invention relates to an iminium salt having the following formula (I) and also to a ruthenium complex having the following formula (II): 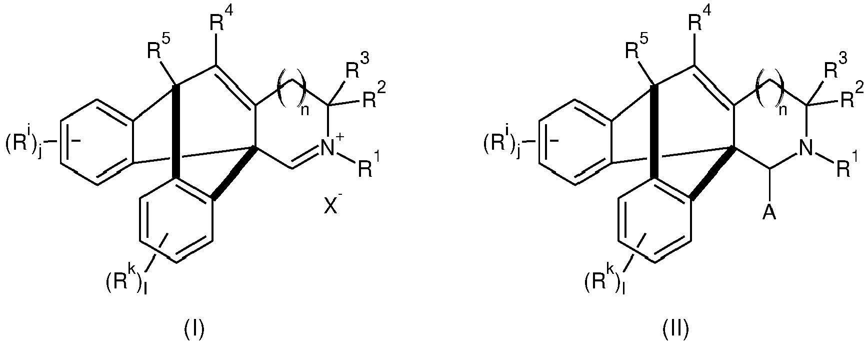 as well as the use of said ruthenium complex as catalyst, in particular in olefin metathesis.

## Description

The present invention concerns iminium salts including a barralene ring, as well as the corresponding ruthenium complexes. The present invention also concerns the use of said complexes as catalysts, in particular in olefin metathesis.

Discovered in the mid of last century, olefin metathesis has become a practical and versatile synthetic tool to efficiently produce carbon-carbon double bonds *(*Handbook of Metathesis, 2nd Edition (Eds.: R. H. Grubbs, A. G. Wenzel, D. J. O'Leary, E. Khosravi); Wiley-VCH: Weinheim, Germany, 2015)**.** Relevant applications were successfully disclosed in various fields such as natural product synthesis (Metathesis in Natural Product Synthesis: Strategies, Substrates, and Catalysts (Eds: Cossy, J.; Arseniyadis, S.; Meyer, C.) Wiley-VCH: Weinheim, Germany, 2010), the transformation of renewable feedstocks (C. Bruneau, C. Fischmeister in Alkene Metathesis for Transformations of Renewables in Organometallics for Green Catalysis (Eds: P. Dixneuf, J. F. Soulé) Topics in Organometallic Chemistry, vol 63. Springer, 2018) or the production of innovative materials (polymers) (S. Kovacic, C. Slugovc, Mater. Chem. Front., 2020, 4, 2235.). This resounding success results from the elaboration of well-defined, air stable and easy to handle ruthenium-arylidene complexes that proved to be highly tolerant towards many organic functionalities *(*O. M. Ogba, N. C. Warner, D. J. O'Leary, R. H. Grubbs, Chem. Soc. Rev. 2018, 47, 4510.). Major improvements in terms of both stability and reactivity of those well-defined ruthenium-arylidene complexes were essentially achieved by the use of specific ancillary ligands such *N*-heterocyclic carbenes (NHCs) (N-Heterocyclic Carbenes: From Laboratory Curiosities to Efficient Synthetic Tools (Eds.: S. Díez-Gonzalez), RSC Catalysis series, RSC Publishing: Cambridge, 2011), or more recently Cyclic (Alkyl)(Amino)Carbenes (CAACs) (J. Morvan, M. Mauduit, G. Bertrand, R. Jazzar, ACS Catal. 2021, 11, 1714.).

However, despite these significant breakthroughs, there is a perpetual requirement for ruthenium olefin metathesis complexes, containing new and easily accessible ancillary ligands, with improved features, which will further allow their use in numerous valuable olefin metathesis applications.

The aim of the present invention is thus to provide new iminium salts as precursors of cyclic aminobarrelene carbene ligands and their use as ancillary ligands for ruthenium olefin metathesis complexes.

Therefore, the present invention relates to an iminium salt having the following formula (I): 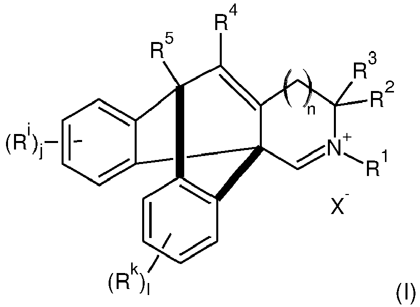 wherein:
- n = 0 or 1;
- R¹ is a (C₆-C₁₄)aryl group, a (C₁-C₆)alkyl group or a (C₈-C₂₀)cycloalkyl group, said aryl group being optionally substituted with at least one substituent chosen from the group consisting of: halogen, (C₁-C₆)alkoxy group, (C₆-C₁₀)aryl group, and (C₁-C₆)alkyl group, said alkyl group being optionally substituted with one or several phenyl group(s) or optionally interrupted by at least one oxygen, nitrogen or sulfur atom, or said alkyl group being optionally substituted with at least one substituent chosen from the group consisting of: (C₆-C₁₆)aryl group, (C₂-C₆)alkenyl group, (C₂-C₆)alkynyl group, hydroxyl, (C₆-C₁₀)arylcarbonyl, (C₁-C₆)alkoxycarbonyl, -C≡C-Si((C₁-C₆)alkyl)₃, and -O-Si((C₁-C₆)alkyl)₃;
   or R¹ is a -NR'ₐR'_{b} group, R'ₐ and R'_{b} being independently from each other selected from the group consisting of: H, (C₁-C₆)alkyl, and (C₆-C₁₀)aryl, or R'ₐ and R'_{b} form together with the nitrogen atom carrying them a N(CH₂)₂₊ₘ heterocyclyl ring, m being 0 or an integer comprised from 1 to 6;
- R² is H, a (C₆-C₁₀)aryl group or a (C₁-C₆)alkyl group;
- R³ is a (C₁-C₆)alkyl group; or R² and R³ may together form, with the carbon atom carrying them, a (C₃-C₆)cycloalkyl;
- R⁴ is selected from the following groups: H, (C₆-C₁₀)aryl, and (C₁-C₆)alkyl,
- R⁵ is selected from the following groups: H, (C₆-C₁₀)aryl, and (C₁-C₆)alkyl,
- j is 0 or an integer comprised from 1 to 4;
- i is an integer varying from 1 to j, and each Rⁱ, identical or different, is selected from the following groups: H, (C₆-C₁₀)aryl, and (C₁-C₆)alkyl,
- l is 0 or an integer comprised from 1 to 4;
- k is an integer varying from 1 to I, and each R^{k}, identical or different, is selected from the following groups: H, (C₆-C₁₀)aryl, and (C₁-C₆)alkyl; and
- X⁻ is a counteranion.

Preferably, in formula (I), n=0. Thus, a preferred group of compounds according to the invention consists of compounds having the following formula (l-1):

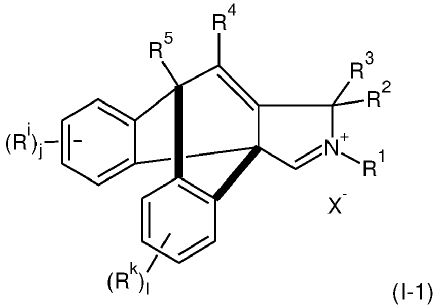

R¹, R², R³, R⁴, R⁵, j, i, l, k, and X⁻ being as defined in formula (l) above.

According to an embodiment, in formula (l) or (l-1), j=l=0.

According to an embodiment, in formula (l) or (l-1), R⁴ is H.

Another preferred group of compounds according to the invention consists of compounds having the following formula (l-2):

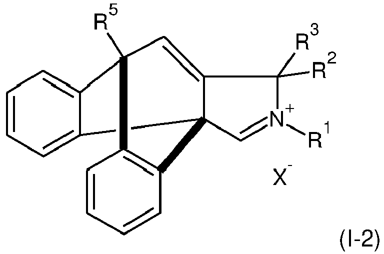

R¹, R², R³, R⁵, and X⁻ being as defined in formula (l) above.

According to an embodiment, in formula (I), (l-1) or (l-2), R² and R³ are (C₁-C₆)alkyl groups, and are preferably identical, and are more preferably methyl groups.

According to an embodiment, in formula (I), (l-1) or (l-2), R² and R³ together form, with the carbon atom carrying them, a (C₃-C₆)cycloalkyl, preferably a cyclohexyl group.

According to an embodiment, in formula (I), (l-1) or (l-2), R⁵ is H.

According to an embodiment, in formula (I), (l-1) or (l-2), R¹ is a (C₆-C₁₄)aryl group, a (C₁-C₆)alkyl group or a (C₈-C₂₀)cycloalkyl group, said aryl group being optionally substituted with at least one substituent chosen from the group consisting of: halogen, (C₁-C₆)alkoxy group, (C₆-C₁₀)aryl group, and (C₁-C₆)alkyl group, said alkyl group being optionally substituted with one or several phenyl group(s) or optionally interrupted by at least one oxygen, nitrogen or sulfur atom, or said alkyl group being optionally substituted with at least one substituent chosen from the group consisting of: (C₆-C₁₆)aryl group, (C₂-C₆)alkenyl group, (C₂-C₆)alkynyl group, hydroxyl, (C₆-C₁₀)arylcarbonyl, (C₁-C₆)alkoxycarbonyl, -C≡C-Si((C₁-C₆)alkyl)₃, and -O-Si((C₁-C₆)alkyl)₃.

According to an embodiment, in formula (I), (l-1) or (l-2), R¹ is a (C₆-C₁₄)aryl group, a (C₁-C₆)alkyl group or a (C₈-C₂₀)cycloalkyl group, said aryl group being optionally substituted with at least one substituent chosen from the group consisting of: halogen, (C₁-C₆)alkoxy group, (C₆-C₁₀)aryl group, and (C₁-C₆)alkyl group, said alkyl group being optionally substituted with one or several phenyl group(s) or optionally interrupted by at least one oxygen, or said alkyl group being optionally substituted with at least one substituent chosen from the group consisting of: (C₆-C₁₆)aryl group, (C₂-C₆)alkenyl group, (C₂-C₆)alkynyl group, hydroxyl, (C₆-C₁₀)arylcarbonyl, (C₁-C₆)alkoxycarbonyl, -C≡C-Si((C₁-C₆)alkyl)₃, and -O-Si((C₁-C₆)alkyl)₃.

According to an embodiment, in formula (I), (l-1) or (l-2), R¹ is a (C₈-C₂₀)cycloalkyl group, such as an an adamantyl group.

According to an embodiment, in formula (I), (l-1) or (l-2), R¹ is a (C₁-C₆)alkyl group, optionally interrupted by at least one oxygen atom, or said alkyl group being optionally substituted with at least one substituent chosen from the group consisting of: (C₆-C₁₆)aryl group, (C₂-C₆)alkenyl group, (C₂-C₆)alkynyl group, hydroxyl, (C₆-C₁₀)arylcarbonyl, (C₁-C₆)alkoxycarbonyl, -C≡C-Si((C₁-C₆)alkyl)₃, and -O-Si((C₁-C₆)alkyl)₃.

According to an embodiment, in formula (I), (l-1) or (l-2), R¹ is a (C₆-C₁₄)aryl group, preferably a phenyl group, optionally substituted with at least one substituent chosen from the group consisting of: halogen, (C₁-C₆)alkoxy group, (C₆-C₁₀)aryl group, and (C₁-C₆)alkyl group.

According to an embodiment, in formula (I), (l-1) or (l-2), R¹ is a (C₆-C₁₄)aryl group, preferably a phenyl group, a (C₁-C₆)alkyl group or a (C₈-C₂₀)cycloalkyl group, said aryl group being optionally substituted with at least one substituent chosen from the group consisting of: halogen, (C₆-C₁₀)aryl group, and (C₁-C₆)alkyl group.

According to an embodiment, in formula (I), (l-1) or (l-2), R¹ is a (C₆-C₁₄)aryl group, preferably a phenyl group, optionally substituted with at least one substituent chosen from the group consisting of: halogen, (C₆-C₁₀)aryl group, and (C₁-C₆)alkyl group.

In the context of the present invention, the expression "Cₜ-C_{z}" means a carbon-based chain which can have from t to z carbon atoms, for example C₁-C₃ means a carbon-based chain which can have from 1 to 3 carbon atoms.

According to the invention, the term "halogen" means: a fluorine, a chlorine, a bromine or an iodine.

According to the invention, the term "alkyl group" means: a linear or branched, saturated, hydrocarbon-based aliphatic group comprising, unless otherwise mentioned, from 1 to 12 carbon atoms. By way of examples, mention may be made of methyl, ethyl, n-propyl, isopropyl, butyl, isobutyl, tert-butyl or pentyl groups;

According to the invention, the term "cycloalkyl group" means: a cyclic carbon-based group comprising, unless otherwise mentioned, from 3 to 12 carbon atoms. By way of examples, mention may be made of cyclopropyl, cyclobutyl, cyclopentyl, cyclohexyl, or adamantyl etc. groups;

According to the invention, the term "haloalkyl group" means: an alkyl group as defined above, in which one or more of the hydrogen atoms is (are) replaced with a halogen atom. By way of example, mention may be made of fluoroalkyls, in particular CF₃ or CHF₂.

According to the invention, the term "alkoxy group" means: an -O-alkyl radical where the alkyl group is as previously defined. By way of examples, mention may be made of -O-(C₁-C₄)alkyl groups, and in particular the -O-methyl group, the -O-ethyl group as -O-C₃alkyl group, the -O-propyl group, the -O-isopropyl group, and as -O-C₄alkyl group, the -O-butyl, -O-isobutyl or -O-tert-butyl group.

According to the invention, the term "aryl group" means: a cyclic aromatic group comprising between 6 and 10 carbon atoms. By way of examples of aryl groups, mention may be made of phenyl or naphthyl groups.

According to the invention, the term "heteroaryl" means: a 5- to 10-membered aromatic monocyclic or bicyclic group containing from 1 to 4 heteroatoms selected from O, S or N. By way of examples, mention may be made of imidazolyl, thiazolyl, oxazolyl, furanyl, thiophenyl, pyrazolyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl and isoquinolinyl groups.

By way of a heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of heteroaryl, of thiophenyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothiophenyl, thienopyridyl, thienopyrimidinyl, pyrrolopyridyl, imidazopyridyl, benzoazaindole, 1,2,4-triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenyl nucleus.

According to the invention, the term "heterocycloalkyl" means: a 4- to 10-membered, saturated or partially unsaturated, monocyclic or bicyclic group comprising from one to three heteroatoms selected from O, S or N; the heterocycloalkyl group may be attached to the rest of the molecule via a carbon atom or via a heteroatom; the term bicyclic heterocycloalkyl includes fused bicycles and spiro-type rings.

By way of saturated heterocycloalkyl comprising from 5 to 6 atoms, mention may be made of oxetanyl, tetrahydrofuranyl, dioxolanyl, pyrrolidinyl, azepinyl, oxazepinyl, pyrazolidinyl, imidazolidinyl, tetrahydrothiophenyl, dithiolanyl, thiazolidinyl, tetrahydropyranyl, tetrahydropyridinyl, dioxanyl, morpholinyl, piperidinyl, piperazinyl, tetrahydrothiopyranyl, dithianyl, thiomorpholinyl or isoxazolidinyl.

When the heterocycloalkyl is substituted, the substitution(s) may be on one (or more) carbon atom(s) and/or on the heteroatom(s). When the heterocycloalkyl comprises several substituents, they may be borne by one and the same atom or different atoms.

The term "alkenyl" as employed herein includes unsaturated, nonaromatic, hydrocarbon groups having 2 to 20 carbons, preferably 2 to 6 carbons, and comprising at least one double bond. Preferably, the alkenyl group is linear. Preferably, the alkenyl group is a -(CH₂)ₙ-C=CH group, n being an integer comprised from 1 to 4.

The term "alkynyl" as employed herein includes unsaturated, nonaromatic, hydrocarbon groups having 2 to 20 carbons, preferably 2 to 6 carbons, and comprising at least one triple bond. Preferably, the alkynyl group is linear. Preferably, the alkynyl group is a -(CH₂)ₙ-C≡CH group, n being an integer comprised from 1 to 4.

The abovementioned "alkyl", "cycloalkyl", "aryl", "heteroaryl" and "heterocycloalkyl" radicals can be substituted with one or more substituents. Among these substituents, mention may be made of the following groups: amino, hydroxyl, thiol, oxo, halogen, alkyl, alkoxy, alkylthio, alkylamino, aryloxy, arylalkoxy, cyano, trifluoromethyl, carboxy or carboxyalkyl.

According to the invention, the term "alkylthio" means: an -S-alkyl group, the alkyl group being as defined above.

According to the invention, the term "arylthio" means: an -S-aryl group, the aryl group being as defined above.

According to the invention, the term "alkylamino" means: an -NH-alkyl group, the alkyl group being as defined above.

According to the invention, the term "cycloalkyloxy" means: an -O-cycloalkyl group, the cycloalkyl group being as defined above.

According to the invention, the term "aryloxy" means: an -O-aryl group, the aryl group being as defined above.

According to the invention, the term "(hetero)arylalkoxy" means: a (hetero)arylalkoxy- group, the (hetero)aryl and alkoxy groups being as defined above.

According to the invention, the term "alkylcarbonyl" means a -CO-alkyl group, the alkyl group being as defined above.

According to the invention, the term "alkoxylcarbonyl" means a -CO-O-alkyl group, the alkyl group being as defined above.

According to the invention, the term "arylcarbonyl" means a -CO-aryl group, the aryl group being as defined above.

According to the invention, the term "aryloxycarbonyl" means a -CO-aryloxy group, the aryloxy group being as defined above.

According to the invention, the term "alkylsulfonyl" means a -SO₂-alkyl group, the alkyl group being as defined above.

According to the invention, the term "arylsulfonyl" means a -SO₂-aryl group, the aryl group being as defined above.

According to the invention, the term "alkylsulfinyl" means a -SO-alkyl group, the alkyl group being as defined above.

According to the invention, the term "arylsulfinyl" means a -SO-aryl group, the aryl group being as defined above.

According to the invention, the term "carboxyalkyl" means: an HOOC-alkyl-group, the alkyl group being as defined above. As examples of carboxyalkyl groups, mention may in particular be made of carboxymethyl or carboxyethyl.

According to the invention, the term "carboxyl" means: a COOH group.

According to the invention, the term "oxo" means: "=O".

When an alkyl radical is substituted with an aryl group, the term "arylalkyl" or "aralkyl" radical is used. The "arylalkyl" or "aralkyl" radicals are aryl-alkyl- radicals, the aryl and alkyl groups being as defined above. Among the arylalkyl radicals, mention may in particular be made of the benzyl or phenethyl radicals.

According to an embodiment, in formula (l) or also in formulae (l-1) or (l-2), X⁻is a counteranion, preferably selected from the group consisting of: BF₄⁻, l⁻, Cl⁻, OTf⁻, Br⁻, PF₆⁻, SbF₆⁻, and B(Ar)₄⁻, Ar representing an aryl group, such as BPh₄⁻. As counteranions the followings may also be mentioned: MXₙ⁻ e.g. CuCl₂⁻, AuBr₂⁻, [Pd(η3-cin)Cl₂]⁻, FeCl₄⁻ (see Ekaterina A. Martynova, Nikolaos V. Tzouras, Gianmarco Pisanò, Catherine S. J. Cazin and Steven P. Nolan (Chemical Communications, 32, 2021)) or [NiCl₄²⁻] (see Mickaël Henrion, Sonía Duarte Barroso, Ana M. Martins, Vincent Ritleng, Michael J. Chetcuti (Polyhedron, volume 87, February 2015, p.398-402) or Yan-Chao Xu, Jie Zhang, Hong-Mei Sun, Qi Shen and Yong Zhang (Dalton Transactions, 23, 2013)).

Any counteranion known from the skilled person may be used. Other examples may be found for example Han Vinh Huynh, Truc Tien Lam and Huyen T. T. Luong (RSC Advances, issue 61, 2018).

According to a preferred embodiment, X⁻ is BF₄⁻, I⁻, OTf⁻, or PF₆⁻,

As preferred iminium salts according to the invention, the compounds having one of the following formulae may be mentioned:

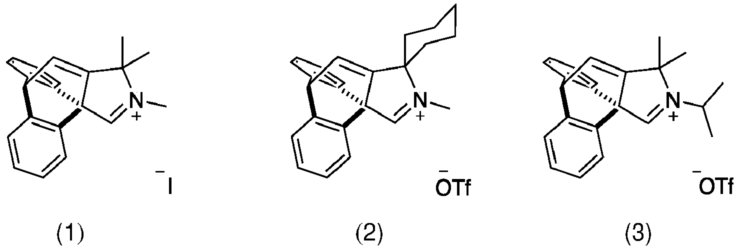

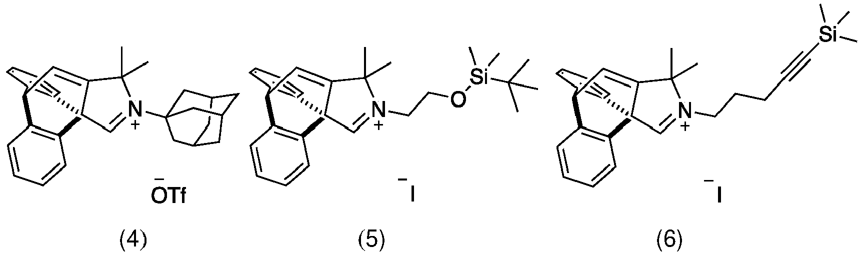

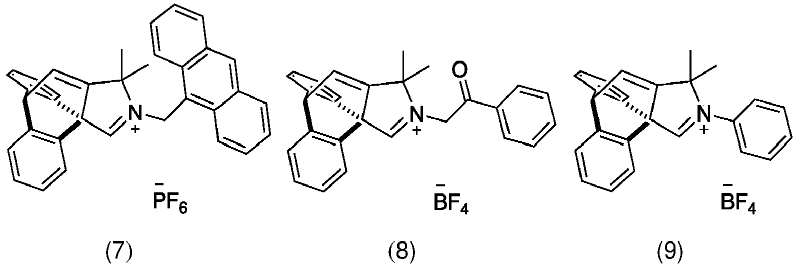

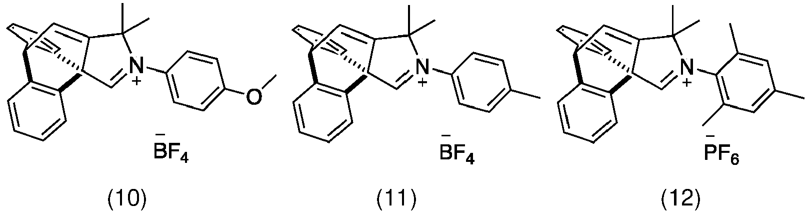

The present invention also relates to a ruthenium complex having the following formula (II): 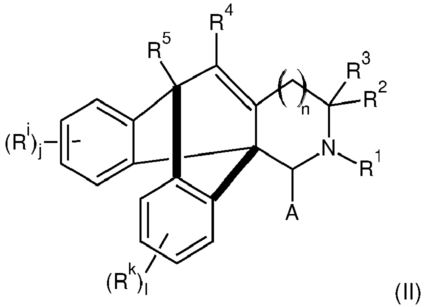 wherein:
- n, j, k, Rⁱ, R^{k}, R¹, R², R³, R⁴ and R⁵ are as defined in formula (I), (l-1) or (l-2) above, and
- A is either a group of formula (1) or a group of formula (2): 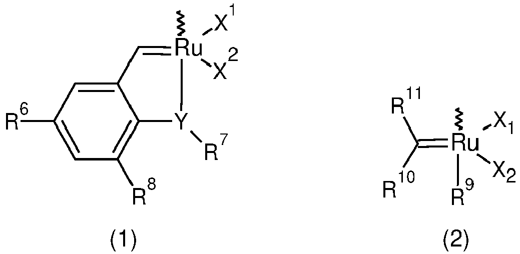 wherein:
- X¹ is an halogen atom, a (C₁-C₆)alkoxy group, a heteroaryl group such as a pyrrole group, or a -S-(C₆-C₁₀)aryl group;
- X² is an halogen atom, a heteroaryl group such as a pyrrole group, or a (C₁-C₆)alkoxy group;
   or X¹ and X² may form together with the ruthenium atom carrying them a heterocycloalkyl group fused with a phenyl group, said phenyl group being possibly substituted with at least one halogen atom;
- Y is an oxygen or a sulfur, preferably an oxygen;
- R⁶ is H or is selected from the following groups: nitro, cyano, (C₁-C₆)alkyl, cycloalkyl, (C₁-C₆)alkoxy, cycloalkyloxy, (C₆-C₁₀)aryl, heteroaryl, (C₆-C₁₀)aryloxy, heteroaryloxy, (C₁-C₆)alkylcarbonyl, arylcarbonyl, (C₁-C₆)alkoxycarbonyl, aryloxycarbonyl, carboxyl, amido, (C₁-C₆)alkylsulfonyl, arylsulfonyl, (C₁-C₆)alkylsulfinyl, arylsulfinyl, (C₁-C₆)alkylthio, arylthio, sulfonamide, halogen, -NRₐR_{b}, -SO₂-NRR', and -N(R_{c})-C(=O)-R_{c},

Rₐ and R_{b} being independently selected from H and (C₁-C₆)alkyl,
R and R' being selected from the following groups: (C₁-C₆)alkyl, (C₆-C₁₀)aryl, heteroaryl, and halo(C₁-C₆)alkyl,
R_{c} being H or being selected from the following groups: (C₁-C₆)alkyl, (C₆-C₁₀)aryl, and heteroaryl,
R_{d} being H or selected from the following groups: (C₁-C₆)alkyl, halo(C₁-C₆)alkyl, cycloalkyl, (C₆-C₁₀)aryl, heteroaryl, (C₁-C₆)alkoxy, cycloalkyloxy, (C₆-C₁₀)aryloxy, and heteroaryloxy;

- R⁷ is a (C₁-C₆)alkyl group or a (C₆-C₁₀)aryl group;
- R⁸ is H, a (C₆-C₁₀)aryl group or a (C₁-C₆)alkyl group;
- R⁹ is a heteroaryl group, or a group -P(R¹²)₃ or P(OR¹²)₃, R¹² being chosen from the group consisting of: (C₁-C₆)alkyl, (C₆-C₁₀)aryl, and cycloalkyl;
- R¹⁰ is a (C₆-C₁₄)aryl group, preferably a phenyl group, said aryl group being optionally substituted with at least one substituent chosen from the group consisting of: halogen, (C₆-C₁₀)aryl group, and (C₁-C₆)alkyl group;
- R¹¹ is H or a (C₆-C₁₄)aryl group, or R¹⁰ and R¹¹ may together form a indenyl group optionally substituted with at least one substituent chosen from the group consisting of: (C₆-C₁₀)aryl group, and (C₁-C₆)alkyl group;

Preferably, in formula (II), n=0. Thus, a preferred group of ruthenium complexes according to the invention consists of ruthenium complexes having the following formula (II-1):

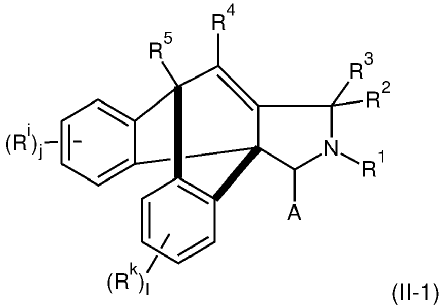

R¹, R², R³, R⁴, R⁵, j, i, l, k, and A being as defined in formula (II) above.

According to an embodiment, in formula (II) or (11-1), j=l=0.

According to an embodiment, in formula (II) or (11-1), R⁴ is H.

Another preferred group of ruthenium complexes according to the invention consists of ruthenium complexes having the following formula (II-2):

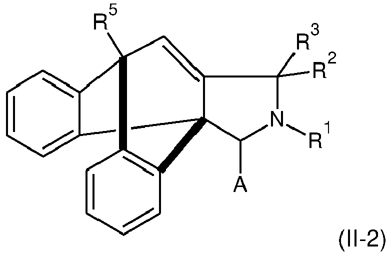

R¹, R², R³, R⁵, and A being as defined in formula (II) above.

According to an embodiment, in formula (II), (II-1) or (II-2), R² and R³ are (C₁-C₆)alkyl groups, and are preferably identical, and are more preferably methyl groups.

According to an embodiment, in formula (II), (II-1) or (II-2), R⁵ is H.

According to an embodiment, in formula (II), (II-1) or (II-2), R¹ is a (C₆-C₁₄)aryl group, a (C₁-C₆)alkyl group or a (C₈-C₂₀)cycloalkyl group, said aryl group being optionally substituted with at least one substituent chosen from the group consisting of: halogen, (C₁-C₆)alkoxy group, (C₆-C₁₀)aryl group, and (C₁-C₆)alkyl group, said alkyl group being optionally substituted with one or several phenyl group(s) or optionally interrupted by at least one oxygen atom, or said alkyl group being optionally substituted with at least one substituent chosen from the group consisting of: (C₆-C₁₆)aryl group, (C₂-C₆)alkenyl group, (C₂-C₆)alkynyl group, hydroxyl, (C₆-C₁₀)arylcarbonyl, (C₁-C₆)alkoxycarbonyl, -C≡C-Si((C₁-C₆)alkyl)₃, and -O-Si((C₁-C₆)alkyl)₃.

According to an embodiment, in formula (II), (II-1) or (II-2), R¹ is a (C₈-C₂₀)cycloalkyl group, such as an an adamantyl group.

According to an embodiment, in formula (II), (II-1) or (II-2), R¹ is a (C₁-C₆)alkyl group, such as an isopropyl group.

According to an embodiment, in formula (II), (II-1) or (II-2), R¹ is a (C₆-C₁₄)aryl group, preferably a phenyl group, optionally substituted with at least one substituent chosen from the group consisting of: halogen, (C₁-C₆)alkoxy group, (C₆-C₁₀)aryl group, and (C₁-C₆)alkyl group.

According to an embodiment, in formula (II), (II-1) or (II-2), R¹ is a phenyl group, optionally substituted with at least one (C₁-C₆)alkyl group. Preferably, R¹ is a phenyl group, substituted with at least one methyl group, and in particular substituted with three methyl groups.

According to an embodiment, in formula (II), (II-1) or (II-2), when A is a group of formula (2), R¹⁰ and R¹¹ together form an indenyl group optionally substituted with at least one substituent chosen from the group consisting of: (C₆-C₁₀)aryl group, and (C₁-C₆)alkyl group.

According to an embodiment, the group of formula (2) has the following formula:

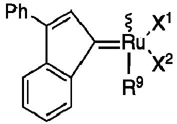

R⁹, X₁ and X₂ being as defined above in formula (II).

According to an embodiment, in formula (II), (II-1) or (II-2), A is a group having the above-mentioned formula (2) wherein X¹ and X¹ are halogen atoms.

Another preferred group of ruthenium complexes according to the invention consists of ruthenium complexes having the following formula (II-3):

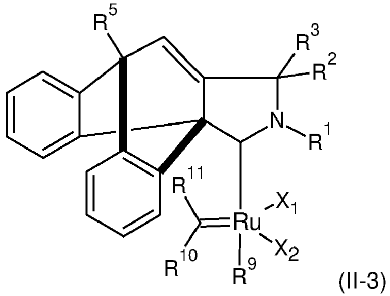

R¹, R², R³, R⁵, R⁹, R¹⁰, R¹¹, X₁, and X₂ being as defined above for formula (II), (II-1) or (II-2).

Preferably, in formula (II-3), X₁ and X₂ are halogen atoms, preferably Cl.

Preferably, in formula (II-3), R¹¹ is H and R¹⁰ is a (C₆-C₁₄)aryl group, preferably a phenyl group, said aryl group being optionally substituted with at least one substituent chosen from the group consisting of: halogen, (C₆-C₁₀)aryl group, and (C₁-C₆)alkyl group. Preferably, in formula (II-3), R¹¹ is H and R¹⁰ is a phenyl group.

Preferably, in formula (II-3), R⁹ is a heteroaryl group, such as a pyridinyl group.

According to an embodiment, in formula (II), (II-1) or (II-2), A is a group having the above-mentioned formula (1). Thus, another preferred group of ruthenium complexes according to the invention consists of ruthenium complexes having the following formula (II-4):

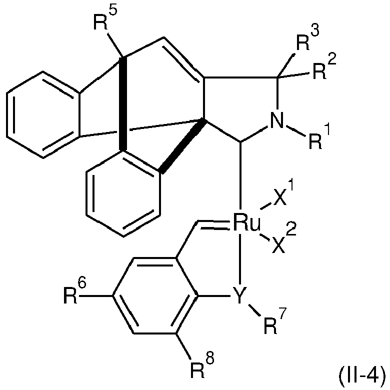

R¹, R², R³, R⁵, R⁶, R⁷, R⁸, Y, X₁, and X₂ being as defined above for formula (II), (II-1) or (II-2).

According to an embodiment, in formula (II-4), Y is an oxygen atom.

According to an embodiment, in formula (II-4), X₁ and X₂ are halogen atoms, preferably Cl.

According to an embodiment, in formula (II-4), X₁ and X₂ are heteroaryl groups, preferably pyrrole groups.

According to an embodiment, in formula (II-4), X₁ and X₂ form together with the ruthenium atom carrying them a heterocycloalkyl group, preferably a cycle with 5 atoms including the ruthenium atom and two sulfur atoms, fused with a phenyl group, said phenyl group being possibly substituted with at least one halogen atom.

According to an embodiment, in formula (II-4), R⁶ is H or nitro.

According to an embodiment, in formula (II-4), R⁸ is H.

According to an embodiment, the ruthenium complex according to the invention has the following formula (III): 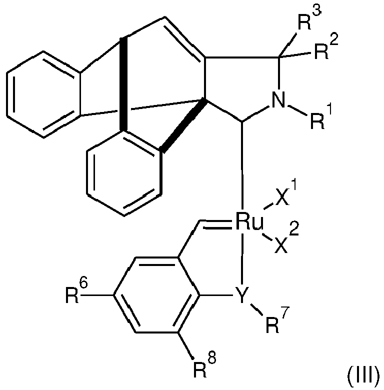 wherein:
- X¹ is an halogen atom or a (C₁-C₆)alkoxy group;
- X² is an halogen atom or a (C₁-C₆)alkoxy group;
- Y is an oxygen or a sulfur, preferably an oxygen;
- R¹ is a (C₆-C₁₀)aryl group, a (C₈-C₂₀)cycloalkyl group or a (C₁-C₆)alkyl group, said aryl group being optionally substituted with at least one substituent chosen from the group consisting of (C₁-C₆)alkyl;
- R² is a (C₆-C₁₀)aryl group or a (C₁-C₆)alkyl group;
- R³ is a (C₁-C₆)alkyl group;
- R⁶ is H, nitro or a (C₁-C₆)alkyl group.
- R⁷ is a (C₁-C₆)alkyl group, such as isopropyl; and
- R⁸ is H, a (C₆-C₁₀)aryl group or a (C₁-C₆)alkyl group.

According to an embodiment, in formula (II), (11-1), (II-2), (II-3) or (II-4) or in formula (III), X₁ and X₂ are halogen atoms, preferably Cl.

According to an embodiment, in formula (II), (11-1), (II-2), (II-3) or (II-4) or in formula (III), R¹ is a phenyl group, optionally substituted with at least one substituent chosen from the group consisting of (C₁-C₆)alkyl, preferably a phenyl group substituted with three alkyl groups, such as methyl groups, or R¹ is a (C₈-C₂₀)cycloalkyl group or a (C₁-C₆)alkyl group.

According to an embodiment, in formula (II), (II-1), (II-2), (II-3) or (II-4) or in formula (III), R² is a (C₁-C₆)alkyl group.

According to an embodiment, in formula (II), (II-1), (II-2), (II-3) or (II-4) or in formula (III), R² and R³ are identical, and are preferably a methyl group.

According to an embodiment, in formula (II), (II-1), (II-2), (II-3) or (II-4) or in formula (III), R⁶ is H or nitro.

As preferred complexes according to the invention, the complexes having one of the following formulae may be mentioned:

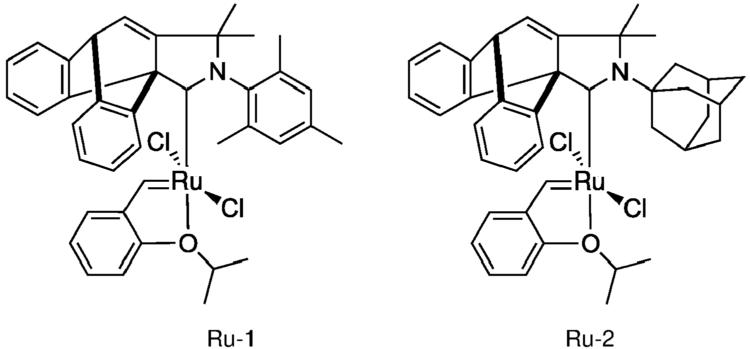

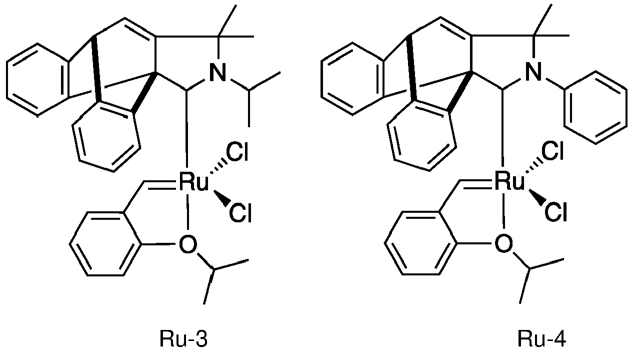

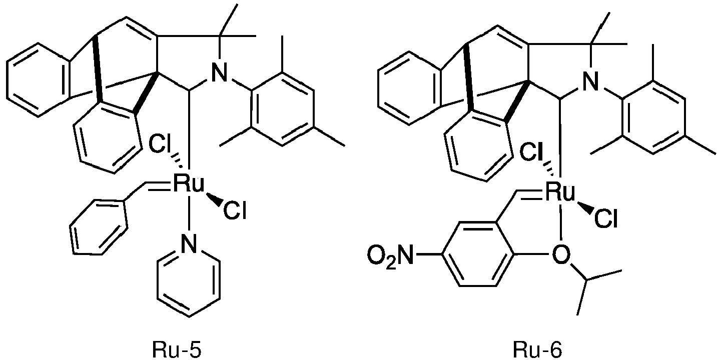

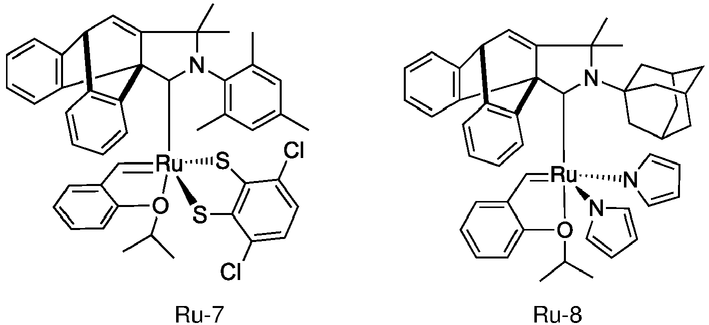

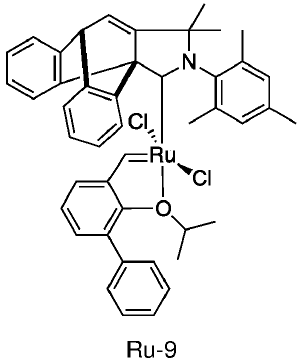

The present invention also relates to the use of the complex according to the invention, preferably having one of the formulae (II), (II-1), (II-2), (II-3), (II-4), or (III) as a catalyst, preferably as a catalyst in olefin metathesis.

### EXAMPLES

### General information

All commercial reagents were used as purchased and without further purification, unless otherwise mentioned. For the synthesis of iminium salts, all reactions were performed under an atmosphere of argon using standard Schlenk techniques; all solvents were dried and degassed using standard procedures. Dichloromethane was dried by distillation from CaH2. Toluene, tetrahydrofuran, dichloromethane and diethyl ether used for complex synthesis and catalysis, were purified using MBraun Solvent Purification Systems. Some reactions were performed in a glove box, under extra dry Argon atmosphere or using standard Schlenk techniques if indicated. Reactions at elevated temperature were maintained by thermostatically controlled oil-baths. A temperature of 0°C was obtained with an ice slush bath and -20°C, -50°C or -78°C were obtained with a mixture of acetone and liquid nitrogen bath. Reactions were monitored by thin-layer chromatography (TLC) carried out on aluminum backed silica gel 60 (F254) plates from MERCK (grain-size distribution 60/20 µm); visualized using 254 nm UV light and KMnO₄ in water for staining. Columns chromatography were performed with silica gel (spherical, particle size 40 µm, neutral) purchased from Sigma-Aldrich. The eluents employed are reported as volume (volume percentages).

NMR: Multinuclear NMR spectra were recorded on a either Bruker Avance 300 MHz, a Varian INOVA 500 MHz (¹H: 500 MHz, ¹³C: 75 MHz) spectrometer, JOEL 400 MHz (¹H: 400 MHz, ¹³C: 101 MHz) or Bruker ARX400 (¹H: 400 MHz, ¹³C: 101 MHz, ³¹P: 162 MHz) spectrometer with complete proton decoupling for nucleus other than ¹H. Chemical shifts are reported in parts per million with the solvent resonance as the internal standard (CDCl₃, ¹H: δ 7.26 ppm, ¹³C: δ 77.16 ppm). Coupling constants (J) are reported in Hertz (Hz). Multiplicities in ¹H NMR are reported using following abbreviations: s = singlet, br s = broad singlet, d = doublet, dd = double doublet, ddd = double double doublet, dt = double triplet, t = triplet, q = quartet, quint = quintet, sept = septet, m = multiplet.

High Resolution Mass Spectrometry (HRMS): For CABCs salts, HRMS were performed at the UC San Diego Mass Spectrometry Laboratory on an Agilent 6230 Accurate-Mass TOFMS spectrometer using electrospray ionization. (ESI). For Ru complexes and catalysis products HRMS were recorded on a Waters QTof-I spectrometer using ESI at the Centre Regional de Mesures Physiques de l'Ouest (CRMPO), Université de Rennes 1.

### PREPARATION OF IMINIUM SALTS

### Imine A

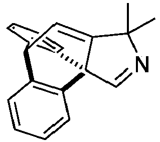

In a Teflon sealed Schlenk, 2-Methyl-3-butyn-2-amine (7.7 mL, 73 mmol) was added to a toluene solution of Anthracene-9-carboxaldehyde (10.00 g, 48 mmol). The mixture was sealed and heated at 120°C overnight. The mixture was then cooled to room temperature and the volatiles were removed under vacuum. The solid was then crystallized in ethyl acetate (200 mL) and Imine **A** was isolated as a colorless solid (9.3 g, 71%).
¹H NMR (500 MHz, CDCl₃): δ = 8.78 (s, 1H), 7.32 (d, ³*J*_{HH} = 6.5 Hz, 2H), 7.25 (d, ³*J*_{HH} = 6.5 Hz, 2H), 6.97 (m, 4H), 7.58 (d, ³*J*_{HH} = 6.0 Hz, 1H), 5.19 (d, ³*J*_{HH} = 6.0 Hz, 1H), 1.25 (s, 6H);
¹³C NMR (75 MHz, CDCl₃): δ 160.90, 146.32, 145.57, 124.85, 124, 53, 123. 30, 120.27, 72.71, 71.80, 52.09, 29.25.
HRMS for **(C₂₀H₁₇N+H⁺)** (M+1⁺): calc.: 272.1434, found: 272.1440.

### Imine B

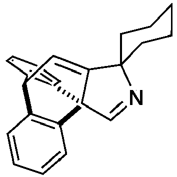

In a Teflon sealed Schlenk, 1-Ethynylcyclohexylamine (9.8 mL, 73 mmol) was added to a toluene solution of Anthracene-9-carboxaldehyde (10 g, 48 mmol). The mixture was sealed and heated at 120 °C overnight. The mixture was then cooled to room temperature and all volatiles were removed under vacuum. The solid residue was then crystallized in ethyl acetate (200 mL). A second crop was crystallized from the mother liquors which is combined another 2.2 g of solid. After combining the 2 batches, Imine **B** was isolated as a colorless solid (12.4 g, 82%).

Data of Imine **B** matches reported values in Ciganek, E. J. Org. Chem. 1980, 45, 1497-1505.

### General procedure for iminium salts from cyclic imines

In a Teflon sealed Schlenk, an alkylation reagent of choice was added to an acetonitrile solution of Imine **A** or **B.** The Schlenk was sealed and heated at 90 °C overnight. After cooling to room temperature, the volatiles were removed under vacuum and the residue was washed with diethyl ether. For an anion exchange, the residue was redissolved in dichloromethane and potassium tetrafluoroborate, potassium hexafluorophosphate, or lithium trifluoromethansulfonate was added and the solution was refluxed at 50°C for 6 hours. After cooling to room temperature, the salt was filtered and washed with diethyl ether (8 x 50 mL).

### Example 1: Preparation of N-methyl CABC A [I] (1)

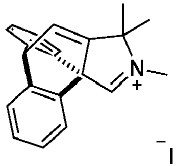

Prepared according to the general procedure for iminium salts using Imine **A** (4.10 g, 15 mmol) and iodomethane (1.6 mL, 26 mmol). Anion metathesis was not performed. Isolated as an off-white solid (5.60 g, 90 % yield).
¹H NMR (500 MHz, CD₃CN): δ = 9.95 (s, 1H), 7.44 (d, ³*J*_{HH} = 6.8 Hz, 2H), 7.39 (d, ³*J*_{HH} = 6.8 Hz, 2H), 7.02-7.11 (m, 5H), 5.47 (d, ³*J*_{HH} = 6.7 Hz, 1H), 3.85 (s, 3H), 1.45 (s, 6H).
¹³C NMR (101 MHz, CD₃CN): δ =178.61, 153.53, 145.03, 143.74, 131.81, 126.99, 125.78, 125.03, 122.44, 78.83, 67.73, 51.92, 39.40.
HRMS for **(C₂₁ H₂₀ N⁺)** (M+.): calc.: 286.1590, found: 286.1589 (-0.3 ppm).

### Example 2: Preparation of N-methyl CABC B [OTf] (2)

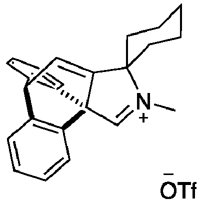

Prepared according to the general procedure for iminium salts using lodomethane (1.25 mL, 20 mmol), Imine **B** (5.00 g, 16 mmol), and lithium trifluoromethanesulfonate (6.20 g, 40 mmol). Isolated as a tan solid (7.10 g, 93% yield).
¹H NMR (500 MHz, CD₃CN): δ = 10.05 (s, 1H), 7.47 (d, ³*J*_{HH} = 6.9 Hz, 2H), 7.42 (d, ³*J*_{HH} = 6.8 Hz, 2H), 7.33 (d, ³*J*_{HH} = 6.0 Hz, 1H), 7.09 (m, 4H), 5.52 (d, ³*J*_{HH} = 6.0 Hz, 1H), 3.93 (s, 3H); 2.03 (t, ³*J*_{HH} = 6.7 Hz, 2H), 1.59 (br d, ³*J*_{HH} = 6.8 Hz, 3H), 1.45 (br q, ³*J*_{HH} = 6.7 Hz, 2H), 1.32 (br d, ³*J*_{HH} = 6.8 Hz, 2H), 1.29 (br d, ³*J*_{HH} = 6.8 Hz, 1H).
¹³C NMR (75 MHz, CD₃CN): δ 178.26, 150.62, 144.76, 143.86, 134.22, 127.08, 125.85, 125.11, 122.15, 82.10, 67.93, 52.25, 39.79, 33.31, 23.90, 23.02.
HRMS for (**C₂₄H₂₄N⁺**) (M⁺): calc.: 326.1903, found: 326.1908.

### Example 3: Preparation of N-isopropyl CABC [OTf] (3)

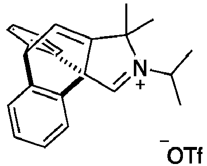

Prepared according to the general procedure for iminium salts using Imine **A** (4.10 g, 15 mmol), 2-bromopropane (1.9 mL, 20 mmol) and lithium trifluoromethanesulfonate (6.2 g, 40 mmol). Isolated as an off-white solid (6.65 g, 95 % yield).
¹H NMR (500 MHz, CD₃CN): δ = 10.51 (s, 1H), 7.48 (d, ³*J*_{HH} = 6.8 Hz, 2H), 7.36 (d, ³*J*_{HH} = 6.8 Hz, 2H), 7.02-7.18 (m, 5H), 5.51 (d, ³*J*_{HH} = 6.7 Hz, 1H), 4.54 (br m, 1H), 1.76 (br m, 6H), 1.53 (s, 6H).
¹³C NMR (75 MHz, CD₃CN): δ 177.47, 152.84, 144.81, 143.65, 131.94, 127.04, 125.97, 125.15, 121.46, 80.83, 67.66, 55.16, 51.81, 26.70, 24.65
HRMS for **(C₂₃H₂₄N⁺)** (M⁺): calc.: 314.1903, found: 314.1913.

### Example 4: Preparation of N-Adamantyl CABC [OTf] (4)

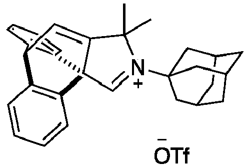

In an aluminum foil covered Schlenk, 1-Bromoadamantane (1.00 g, 4.648 mmol) and 2,6-Di-*tert*-butyl-4-methylpyridine (0.125 g, 0.609 mmol) were dissolved in 15 mL of hexanes. To this solution, silver triflate (1.40 g, 5.438 mmol) was added and the reaction was stirred overnight at room temperature. The mixture was then cannula filtered under an inert atmosphere into a new Schlenk containing imine **1** with 3 x 15 mL extractions of hexanes. The reaction was stirred for another 24 hours at room temperature to yield a pink suspension. The suspension was dissolved in DCM, filtered, and concentrated. The solid was washed with Et₂O and then set for crystallization in DCM by slow diffusion of Et₂O. The resultant white crystals were filtered and washed with Et₂O (1.6580 g, 64%).
¹H NMR (400 MHz, CD₃CN) δ 10.41 (s, 1H), 7.47 (dd, *J* = 6.9, 1.5 Hz, 2H), 7.36 (dd, *J* = 7.1, 1.5 Hz, 2H), 7.12 (td, *J* = 7.4, 1.5 Hz, 2H), 7.07 (td, *J* = 7.4, 1.5 Hz, 2H), 7.02 (dd, *J* = 6.1, 0.9 Hz, 1H), 5.50 (d, *J* = 6.1 Hz, 1H), 2.51 (d, *J* = 3.0 Hz, 6H), 1.84 (s, 6H), 1.73 (s, 6H).
¹³C NMR (101 MHz, CD₃CN) δ 186.23, 152.30, 144.79, 143.86, 142.80, 134.58, 132.54, 131.43, 127.36, 126.37, 125.60, 121.44, 86.82, 68.37, 52.00, 27.80, 20.92, 19.83.
HRMS for **(C₃₀ H₃₂ N⁺)** (M+.): calc: 406.2529, found: 406.2525 (-1.0 ppm).

### Example 5: Preparation of N-2-OtBDMS ethane CABC [I] (5)

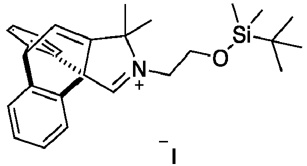

Prepared according to the general procedure for iminium salts using Imine **A** (4.10 g, 15 mmol), tert-butyl(2-iodoethoxy)-dimethylsilane (5.70 g, 20 mmol). Anion metathesis was not performed. Isolated as an off-white solid (7.30 g, 87% yield).
¹H NMR (500 MHz, CDCl₃): δ = 10.96 (s, 1H), 7.65 (d, ³*J*_{HH} = 6.3 Hz, 2H), 7.37 (d, ³*J*_{HH} = 6.8 Hz, 2H), 6.95-7.05 (m, 5H), 5.36 (d, ³*J*_{HH} = 6.6 Hz, 1H), 4.72 (br m, 1H), 4.39 (br m Hz, 1H), 1.48 (s, 6H), 0.92 (s, 9H), 0.18 (m, 6H).
¹³C NMR (75 MHz, CDCl₃): δ 177.11, 152.44, 143.25, 142.38, 130.66, 126.14, 125.46, 124.00, 122.23, 78.63, 67.57, 61.53, 52.89, 51.54, 26.86, 25.88, 18.44, -5.10
HRMS for **(C₂₈H₃₆NOSi⁺)** (M⁺): calc.: 430.2561, found: 340.2075.

### Example 6: Preparation of N-5-(trimethylsilyl)-4-pentynyl CABC [I] (6)

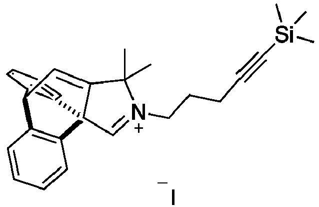

Prepared according to the general procedure for iminium salts using Imine **A** (4.10 g, 15 mmol), (5-iodo-1-pentynyl)trimethylsilane (2.4 mL, 20 mmol). Anion metathesis was not performed. Isolated as an off-white solid (6.50 g, 92% yield).
¹H NMR (500 MHz, CD₃CN): δ = 10.67 (s, 1H), 7.57 (d, ³*J*_{HH} = 6.9 Hz, 2H), 7.47 (d, ³*J*_{HH} = 6.9 Hz, 2H), 7.03-7.10 (m, 5H), 5.52 (d, ³*J*_{HH} = 6.6 Hz, 1H), 4.28 (t, ³*J*_{HH} = 6.5 Hz, 2H), 2.57 (t, ³*J*_{HH} = 6.5 Hz, 2H), 2.42 (q, ³*J*_{HH} = 6.5 Hz, 2H), 1.52 (s, 6H), 0.17 (m, 9H).
¹³C NMR (75 MHz, CD₃CN): δ 177.72, 153.31, 144.93, 143.71, 131.73, 126.94, 125.80, 125.03, 122.41, 105.35, 87.31, 80.18, 67.85, 51.88, 50.30, 28.36, 26.80, 17.18, -0.04.
HRMS for **(C₂₈H₃₂NSi⁺)** (M⁺): calc.: 410.2299, found: 410.2311.

### Example 7: Preparation of N-9-methylanthracene CABC [PF₆] (7)

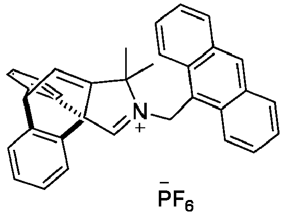

Prepared according to the general procedure for iminium salts using Imine **A** (4.10 g, 15 mmol), 9-(bromomethyl)anthracene (5.40 g, 20 mmol), and potassium hexafluorophosphate (5.50 g, 30 mmol). Isolated as an off-white solid (8.20 g, 89% yield).
¹H NMR (500 MHz, CD₃CN): δ = 9.24 (s, 1H), 8.98 (s, 1H), 8.33 (d, ³*J*_{HH} = 7.0 Hz, 2H), 8.17 (d, ³*J*_{HH} = 7.1 Hz, 2H), 7.91 (dd, ³*J*_{HH} = 7.0, 2.1 Hz, 2H), 7.82 (dd, ³*J*_{HH} = 7.1, 1.6 Hz, 2H), 7.40 (d, ³*J*_{HH} = 6.8 Hz, 2H), 7.12 (d, ³*J*_{HH} = 6.5 Hz, 1H), 7.03 (m, 2H), 6.82 (m, 2H), 6.54 (d, ³*J*_{HH} = 6.9 Hz, 2H), 6.20 (s, 2H), 5.54 (d, ³*J*_{HH} = 6.5 Hz, 1H), 1.89 (s, 6H).
¹³C NMR (75 MHz, CD₃CN): δ 178.31, 153.66, 144.74, 143.41, 133.02, 132.79, 132.27, 132.13, 130.89, 129.76, 126.99, 126.89, 125.68, 125.12, 122.96, 121.36, 119.59, 81.11, 67.13, 51.87, 48.58, 27.20.
HRMS for **(C₃₅H₂₈N⁺)** (M⁺): calc.: 462.2216, found: 462.2233.

### Example 8: Preparation of N-acetophenone CABC [BF₄] (8)

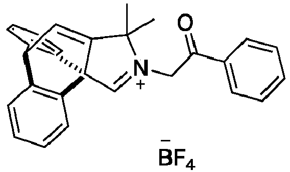

Prepared according to the general procedure for iminium salts using Imine **B** (4.10 g, 15 mmol), 2-bromoacetophenone (4.00 g, 20 mmol), and potassium tetrafluoroborate (3.80 g, 30 mmol). Isolated as an off-white solid (6.60 g, 91 %).
¹H NMR (500 MHz, CD₃CN): δ = 10.25 (s, 1H), 8.19 (d, ³*J*_{HH} = 6.6 Hz, 2H), 7.82 (d, ³*J*_{HH} = 6.7 Hz, 1H), 7.67 (d, ³*J*_{HH} = 6.6 Hz, 2H), 7.60 (br d, ³*J*_{HH} = 6.8 Hz, 2H), 7.49 (br d, ³*J*_{HH} = 6.9 Hz, 2H), 7.10-7.18 (m, 5H), 6.00 (s, 2H), 5.54 (d, ³*J*_{HH} = 6.9 Hz, 1H), 1.42 (m, 6H).
¹³C NMR (75 MHz, CD₃CN): δ 188.91, 183.02, 152.87, 144.86, 143.82, 136.34, 133.67, 132.04, 130.13, 129.78, 127.01, 125.89, 125.07, 122.22, 80.12, 68.40, 57.20, 51.92, 25.89.
HRMS for **(C₂₈H₂₄NO⁺)** (M⁺): calc.: 390.1852, found: 390.1867.

### Example 9: Preparation of N-Phenyl CABC [BF₄] (9)

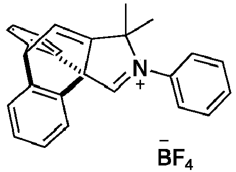

In a Schlenk, imine **A** (0.3002 g, 1.106 mmol) was mixed with diphenyliodonium triflate (0.4755 g, 1.106 mmol), and Cu(OAc)₂ (0.011 g, 0.055 mmol) in 7 mL DMF for 24 hours at 100°C. After removal of solvent, the solid was resuspended in acetonitrile and passed over decolorizing charcoal to yield a clear blue solution that was crystallized by slow diffusion of diethyl ether into acetonitrile. White crystals were washed with diethyl ether and isolated (0.4852 g, 88%). Further purification can be obtained by a recrystallization by slow diffusion of diethyl ether into dichloromethane.
¹H NMR (400 MHz, CD₃CN)δ 10.32 (s, 1H), 7.87 - 7.80 (m, 1H), 7.80 - 7.70 (m, 2H), 7.74 - 7.67 (m, 2H), 7.56 - 7.46 (m, 4H), 7.21 (dd, *J* = 6.0, 1.0 Hz, 1H), 7.13 (pd, *J* = 7.7, 1.5 Hz, 4H), 5.58 (d, *J* = 6.1 Hz, 1H), 1.52 (s, 6H).
¹³C NMR (101 MHz, CD₃CN) δ 182.54, 152.43, 144.86, 143.73, 133.53, 132.60, 131.01, 127.26, 126.96, 126.05, 125.32, 122.18, 83.28, 68.04, 51.96, 27.53.
HRMS for **(C₂₆ H₂₂ N⁺)** (M+.): calc.: 348.1747, found: 348.1744 (-0.9 ppm).

### Example 10: Preparation of N-4-Anisyl CABC [BF₄] (10)

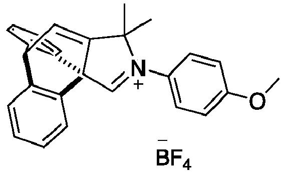

In a Schlenk, imine **1** (0.1001 g, 0.368 mmol) was mixed with (p-anisyl)-2,4,6-triisopropylphenyl iodonium tetrafluoroborate (0.1933, 0.368 mmol), and Cu(OAc)₂ (0.0034 g, 0.018 mmol) in 6 mL DMF for 24 hours at 110 °C. After removal of solvent, the solid was resuspended in acetonitrile and passed over decolorizing charcoal to yield a clear lavender solution that was crystallized by slow diffusion of diethyl ether into acetonitrile. Beige crystals were isolated and washed with diethyl ether (0.1118 g, 65%). Further purification can be obtained by a recrystallization by slow diffusion of diethyl ether into dichloromethane.
¹H NMR (500 MHz, CD₃CN) δ 10.24 (s, 1H), 7.67 - 7.58 (m, 2H), 7.51 (ddd, *J* = 7.1, 2.9, 1.5 Hz, 4H), 7.28-7.21 (m, 2H), 7.19 (dd, *J* = 6.0, 1.0 Hz, 1H), 7.17 - 7.06 (m, 4H), 5.56 (d, *J* = 6.1 Hz, 1H), 3.93 (s, 3H), 1.51 (s, 6H).
¹³C NMR (101 MHz, CD₃CN) δ 181.67, 163.25, 152.50, 144.74, 143.61, 132.48, 132.16, 128.34, 128.21, 127.19, 126.89, 126.02, 125.71, 125.20, 125.01, 122.16, 121.97, 115.87, 115.58, 82.79, 67.61, 56.64, 56.46, 51.82, 51.68, 27.46, 27.30.
HRMS for **(C₂₇ H₂₄ N O⁺)** (M+.): calc.: 378.1852, found: 378.1851 (-0.3 ppm).

### Example 11: Preparation of N-4-Tolyl CABC [BF₄] (11)

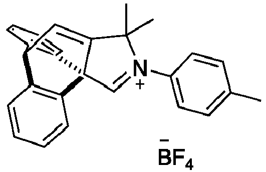

In a Schlenk, imine 1 (0.2000 g, 0.737 mmol) was mixed with di(p-tolyl)iodonium triflate (0.5067 g , 1.106 mmol), and Cu(OAc)₂ (0.0090 g, 0.050 mmol) in 3 mL of DMF at 100°C for 14.5 hours. The solvent was then removed under vacuum and the mixture was stirred vigorously in NH₄OH and extracted 3x with dichloromethane. The combined organic layers were washed 3x with NH₄OH, followed by brine. The organic layer was dried with MgSO₄ and concentrated to an orange oil. The oil was then resuspended in diethyl ether and HCI Et₂O (0.45 mL 2M, 1.22 equiv) was added to the solution. The precipitate was filtered, washed with diethyl ether, and resuspended in a warm aqueous solution with spare acetone. A warm aqueous solution of NaBF₄ (0.18 g, 2 equiv) was slowly added and a precipitate evolved. After stirring overnight, a fluffy white precipitate was filtered and washed with diethyl ether (0.20 g, 0.445 mmol, 60%)
¹H NMR (400 MHz, CD₃CN) δ 10.24 (s, 1H), 7.56 (s, 4H), 7.52 - 7.46 (m, 4H), 7.20 (d, *J* = 6.1 Hz, 1H) 7.17 - 7.06 (m, 4H), 5.56 (d, *J* = 6.1 Hz, 1H) 2.52 (s, 3H), 1.51 (s, 6H).
¹³C NMR (101 MHz, CD₃CN) δ 182.54, 152.59, 144.91, 144.87, 144.48, 143.77, 132.54, 131.40, 127.25, 126.70, 126.05, 125.29, 122.17, 83.06, 67.92, 51.95, 27.57, 21.27.
HRMS for **(C₂₇H₂₄ N⁺)** (M+.): calc.: 362.1903, found: 362.1904 (0.3 ppm).

### Example 12: Preparation of N-Mesityl CABC [PF₆] (13)

### N-Mesityl dimethyl propargylamine

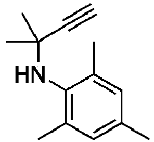

A solution of Cu⁰ (0.05 g, 2 mol%), CuCI (0.5 g, 14 mol%), 2,4,6-trimethylaniline (5.0 mL, 35.5 mmol) and triethylamine (6.9 mL, 50 mmol) in dioxanes was cooled to 0 °C and stirred vigorously. To this solution 3-chloro-3-methyl-1-butyne (5.5 mL, 53 mmol) was added dropwise. The mixture was warmed to room temperature overnight. The red mixture was then concentrated under reduced pressure and extracted with pentanes. The combined organic layer was concentrated under reduced pressure and then distilled under vacuum at 115 °C to yield an orange oil (3.4 g, 48%)
¹H NMR (500 MHz, CDCl₃) δ 6.87 (s, 2H), 2.37 (s, 6H), 2.26 (s, 4H), 1.50 (s, 6H).
¹³C NMR (75 MHz, CDCl₃) δ 139.96, 135.29, 133.59, 129.32, 90.14, 70.27, 52.23, 31.34, 20.86, 20.17.

### 9-(N-Mesityl dimethyl propargyl methylamine) anthracene

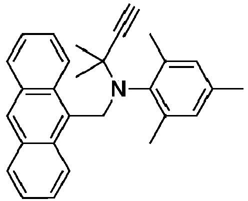

In a Schlenk, Kl (0.21 g, 1.84 mmol) and K₂CO₃ (14.28 g, 103.32 mmol) were heated under vacuum. To this mixture, 9-(Bromomethyl)anthracene (2.00 g, 7.38 mmol) and alkyne (1.49 g, 7.38 mmol) were added and dissolved in dichloromethane (12 mL). The mixture was stirred at room temperature for 2 days. The mixture was then concentrated and extracted with Dl H₂O and Et₂O (3x 50 mL). The combined organic layer was washed with brine (100 mL) and dried with MgSO₄. The solution was concentrated under reduced pressure at 25 °C to yield a yellow solid. The mixture, containing 45% substituted product by NMR, was used immediately without purification.
¹H NMR (500 MHz, CDCl₃,) δ 8.39 (d, *J=* 8.4 Hz, 2H), 8.35 (s, 1H), 7.93 (d, *J*= 8.4 Hz, 2H), 7.43-7.29 (m, 6H), 2.77 (s, 1H), 6.68 (s, 2H), 5.46 (s, 2H), 2.77 (s, 1H), 2.18 (s, 3H), 2.11 (s, 6H), 1.30 (s, 6H).

### Preparation of N-Mesityl CABC [PF₆] (12)

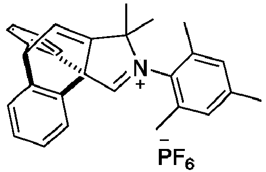

To an ethereal solution of the substituted anthracene, DDQ (1.0 equiv) was added at room temperature. A dark precipitate formed and the mixture was stirred overnight. The mixture was then filtered and washed with ether (3 x 50 mL). The solid was resuspended in toluene and heated at 90 °C for two hours. After concentration under reduced pressure, the green solid was reacted with excess KOH in Dl H₂O and extracted with pentane (4 x 50 mL). The combined organic layer was concentrated and resuspended in diethyl ether. HCI Et₂O (2.0 equiv) was added slowly to the solution and a beige precipitate was filtered and washed with Et₂O. The solid was then dissolved in warm Dl H₂O and a solution of KPF₆ (3.0 equiv) in warm Dl H₂O was slowly added. A beige precipitate formed and was filtered and washed with diethyl ether. The solid was then crystallized by slow diffusion of diethyl ether into dichloromethane. White crystals were obtained, filtered, washed with diethyl ether, and then dried under vacuum (0.74 g, 18% from 9-(bromomethyl)anthracene).
¹H NMR (500 MHz, CD₃CN) δ 10.33 (s, 1H), 7.54 (d, *J* = 7.0 Hz, 2H), 7.48 (d, *J* = 7.0 Hz, 2H), 7.27 (s, 2H), 7.16 (m, 5H), 5.60 (d, *J* = 6.1 Hz, 1H), 2.41 (s, 3H), 2.30 (s, 6H), 1.50 (s, 6H).
¹³C NMR (101 MHz, CD₃CN) δ 186.23, 152.30, 144.79, 143.86, 142.80, 134.58, 132.54, 131.43, 127.36, 126.37, 125.60, 121.44, 86.82, 68.37, 52.00, 27.80, 20.92, 19.83.
HRMS for **(C₂₉ H₂₈ N⁺)** (M+.): calc.: 390.2216, found: 390.2215 (-0.3 ppm).

### PREPARATION OF COMPLEXES

### General procedure for complexes of room temperature stable carbenes

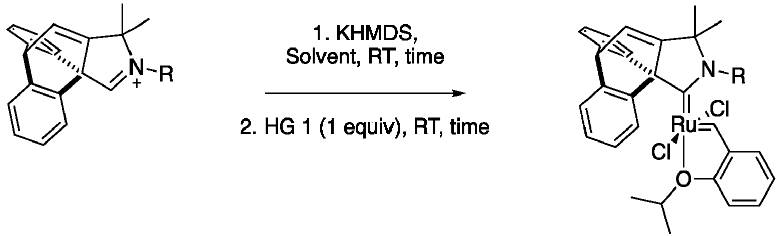

**General Procedure:** In a glove box, CABC salt (1.5 equiv) was dissolved in dry and degassed solvent of choice (10 mL/mmol Ru). KHMDS (1.6 equiv) was added. The mixture was allowed to stir for the indicated time at RT. To this mixture, Hoveyda-Grubbs 1^{st} generation complex (HG1) (Dichloro(2-isopropoxybenzylidene) (tricyclohexylphosphine) ruthenium(II) purchased from Umicore) (1 equiv) was added and the mixture was stirred the indicated time at RT. The solvent was removed under vacuum and the product was purified by column chromatography (eluent: toluene).

The solid was then diluted in the minimum amount of DCM and precipitated in Hexane.

### Example 13: Preparation of N-Mesityl CABC Hoveyda type Ru complex (Ru-1) [JAT-157]

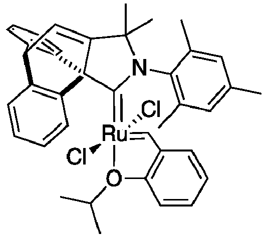

Ru-1 was prepared according to general procedure for the room temperature stable carbenes complexes synthesis with *N*-Mesityl CABC **[PF₆]** (106.7mg, 0.2 mmol, 1.3 equiv), THF (2 mL), KHMDS (39.7 mg, 0.2 mmol, 1.3 equiv), and HG-1 complex (95.7 mg, 0.16 mmol, 1 equiv). The salt was deprotonated during 5 minutes at RT, followed by 2h of stirring with HG1 at RT. The desired product was obtained after purification (eluent: toluene) as a green solid (92 mg, 78 % yield).
¹H NMR (400 MHz, CDCl₃) δ 18.20 (s, 1H), 8.05 (dd, *J* = 7.7, 1.1 Hz, 2H), 7.59 (ddd, *J* = 8.8, 7.3, 1.7 Hz, 1H), 7.43 (dd, *J* = 7.3, 1.3 Hz, 2H), 7.22 (dd, *J* = 7.7, 1.6 Hz, 1H), 7.19 (s, 2H), 7.07 - 6.98 (m, 3H), 6.86 (dtd, *J* = 14.0, 7.5, 1.1 Hz, 3H), 6.78 (d, *J* = 6.0 Hz, 1H), 5.30 (d, *J* = 6.0 Hz, 1H), 5.15 (sept, *J* = 6.1 Hz, 1H), 2.47 (s, 6H), 2.43 (s, 3H), 1.55 (d, *J* = 6.1 Hz, 7H), 1.36 (s, 6H).
¹³C NMR (101 MHz, CDCl₃) δ 297.5 and 297.2 (d, 1C), 266.8, 159.3, 154.8, 147.2, 145.3, 143.6, 143.6, 139.3, 138.9, 135.7, 131.4, 130.5, 127.6, 126.3, 124.7, 124.6, 124.1, 123.8, 122.6, 121.8, 113.3, 75.6, 75.4, 52.0, 35.7, 31.6, 30.6, 24.9, 21.5, 20.9.
HRMS for **(C₃₉ H₃₉ N O ³⁵Cl₂ ¹⁰²Ru)** (M+.): calc.: 709.14467, found: 709.1449 (0 ppm).

### Example 14: Preparation of N-Adamantyl CABC Hoveyda type Ru complex (Ru-2) [JAT-168]

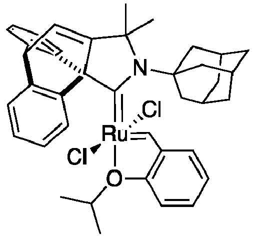

Ru-2 was prepared according to general procedure for the room temperature stable carbenes complexes synthesis with *N*-Adamantyl CABC [OTf] (198 mg, 0.487 mmol, 1.5 equiv), toluene (2 mL), KHMDS (108 mg, 0.541 mmol, 1.6 equiv), and HG-1 complex (200 mg, 0.333 mmol, 1 equiv). The salt was deprotonated during 30 minutes at RT, followed by 2h of stirring with HG1 at RT. The desired product was obtained after purification (eluent: toluene) as a green solid (179.7 mg, 74 % yield).
¹H NMR (400 MHz, CDCl₃) δ 18.52 (s, 1H), 8.23 - 8.17 (m, 2H), 7.64 (ddd, *J* = 8.7, 7.2, 1.7 Hz, 1H), 7.37 (dd, *J* = 7.3, 1.4 Hz, 2H), 7.15 (d, *J* = 8.5 Hz, 1H), 7.05 (dd, *J* = 7.6, 1.7 Hz, 1H), 6.98 (td, *J* = 7.4, 1.1 Hz, 2H), 6.88 (td, *J* = 7.4, 0.8 Hz, 1H), 6.78 (td, *J* = 7.6, 1.4 Hz, 2H), 6.65 (d, *J* = 6.0 Hz, 1H), 5.31 (sept, *J* = 6.2 Hz, 1H), 5.21 (d, *J* = 6.0 Hz, 1H), 3.28 (s, 6H), 2.11 (d, *J* = 12.4 Hz, 3H), 1.91 (d, *J* = 6.1 Hz, 8H), 1.86 (d, *J* = 3.0 Hz, 1H), 1.61 (s, 6H).
¹³C NMR (101 MHz, CDCl₃) δ 312.86 and 312.67 (d, 1C), 259.47, 161.14, 155.51, 147.77, 145.15, 143.54, 132.48, 127.10, 125.25, 124.63, 124.48, 124.24, 122.22, 122.04, 113.56, 75.63, 74.96, 74.48, 66.61, 51.88, 35.67, 33.49, 30.95, 22.64.
HRMS for **(C₄₀ H₄₃ N O ³⁵Cl₂ ¹⁰²Ru)** (M+.): calc.: 725.17597, found : 725.1758 (0 ppm).

### General procedure for complexes of low temperature stable carbenes

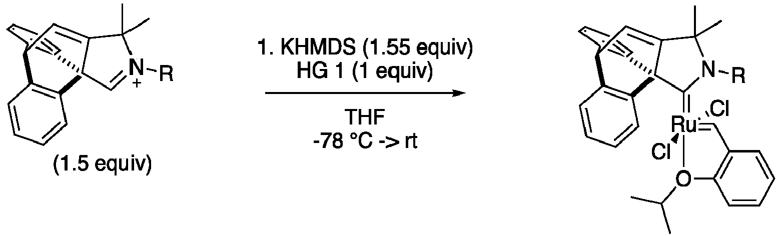

**General Procedure**: In a glove box, an oven-dried Schlenk was charged with CABC salt (1.5 equiv), KHMDS (1.55 equiv) and Hoveyda-Grubbs 1^{st} generation complex (HG1) (Dichloro(2-isopropoxybenzylidene)(tricyclohexylphosphine) ruthenium(II) purchased from Umicore) (1 equiv). The Schlenk was then connected to the argon line and cooled to -78°C. Precooled, dry, and degassed THF was cannulated to the Schlenk, and the mixture was stirred for the indicated time and warmed up slowly from -78°C to RT overnight.

The solvent was removed under vacuum and the product was purified by column chromatography (eluent: pentane/acetone 9:1). The solid was then diluted in the minimum amount of DCM and precipitated with Hexane.

### Example 15: Preparation of N-isopropyl CABC Hoveyda type Ru complex (Ru-3) [JM-659]

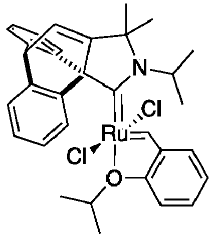

Ru-3 was prepared according to general procedure for the low temperature stable carbenes complexes synthesis with *N*-isoPropyl CABC [BF₄] (102.3 mg, 0.244 mmol, 1.5 equiv), THF (1.5 mL), KHMDS (56.0 mg, 0.281 mmol, 1.6 equiv), and HG-1 complex (102.5 mg, 0.171 mmol, 1 equiv). The desired product was obtained after purification (eluent: pentane/acetone 9:1) as a brown solid (27.7 mg, 26 % yield).
¹H NMR (400 MHz, CDCl₃) δ 18.05 (s, 1H), 7.85 (dd, *J* = 7.7, 1.1 Hz, 2H), 7.62 (ddd, *J* = 8.7, 7.3, 1.7 Hz, 1H), 7.38 (dd, *J* = 7.3, 1.3 Hz, 2H), 7.25 (dd, *J* = 7.6, 1.6 Hz, 1H), 7.17 (d, *J* = 8.4 Hz, 1H), 7.03 - 6.90 (m, 3H), 6.81 (td, *J* = 7.6, 1.3 Hz, 2H), 6.67 (d, *J* = 6.0 Hz, 1H), 5.99 (h, *J* = 6.7 Hz, 1H), 5.40 (sept, *J* = 6.2 Hz, 1H), 5.24 (d, *J* = 6.0 Hz, 1H), 2.05 - 1.94 (m, 12H), 1.51 (s, 6H).
¹³C NMR (101 MHz, CDCl₃) δ 295.06 and 294.66 (d, 1C), 258.22, 161.05, 154.65, 146.47, 145.27, 142.76, 142.73, 131.05, 126.68, 124.61, 124.56, 124.06, 123.46, 122.54, 122.14, 113.23, 75.17, 73.74, 73.29, 62.37, 51.76, 31.76, 24.89, 22.24.
HRMS for **(C₃₆ H₃₃ N O ³⁵Cl₂ ¹⁰²Ru)** (M+.): calc.: 633.11337, found: 667.1135 (0 ppm).

### Example 16: Preparation of N-Phenyl CABC Hoveyda type Ru complex (Ru-4) [JAT-150]

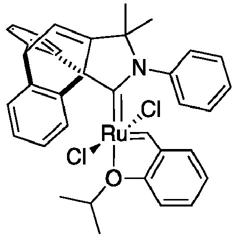

Ru-4 was prepared according to general procedure for the low temperature stable carbenes complexes synthesis with *N*-Phenyl CABC [BF₄] (217.4 mg, 0.500 mmol, 1.5 equiv), THF (2 mL), KHMDS (106.3 mg, 0.533 mmol, 1.6 equiv), and HG-1 complex (199.8 mg, 0.333 mmol, 1 equiv). The reaction time was modified: after the addition of THF in the mixture, the reaction was stirred for one hour during which time the temperature increased from -78°C to approximately -50°C, before removal from the acetone bath. The mixture was stirred for one more hour at RT. The desired product was obtained after purification (eluent: toluene) as a brown solid (127.6 mg, 62 % yield).
¹H NMR (400 MHz, CDCl₃) δ 18.22 (s, 1H), 7.99 (dt, *J* = 7.7, 1.5 Hz, 4H), 7.70 - 7.54 (m, 4H), 7.44 (dd, *J* = 7.4, 1.3 Hz, 2H), 7.28 - 7.21 (m, 1H), 7.10 - 6.97 (m, 3H), 6.95 - 6.81 (m, 4H), 5.32 (d, *J* = 6.0 Hz, 1H), 5.21 (sept, *J* = 6.2 Hz, 1H), 3.76 (s, 2H), 1.52 (s, 6H), 1.34 (s, 7H).
¹³C NMR (101 MHz, CDCl₃) δ 298.45 and 298.24 (d, 1C), 262.14, 158.44, 154.75, 146.65, 145.23, 143.13, 137.11, 134.11, 131.25, 129.78, 128.75, 128.42, 127.97, 124.76, 124.72, 124.01, 123.25, 122.76, 122.69, 122.05, 113.19, 75.52, 74.78, 73.34, 51.93, 43.47, 29.31, 21.56.
HRMS for **(C₃₆ H₃₃ N O ³⁵Cl₂ ¹⁰²Ru)** (M+.): calc.: 667.09772, found: 667.0979 (0 ppm).

### Example 17: Preparation of N-Mesityl CABC CHPh Pyridine Ru complex (Ru-5) [JAT-148]

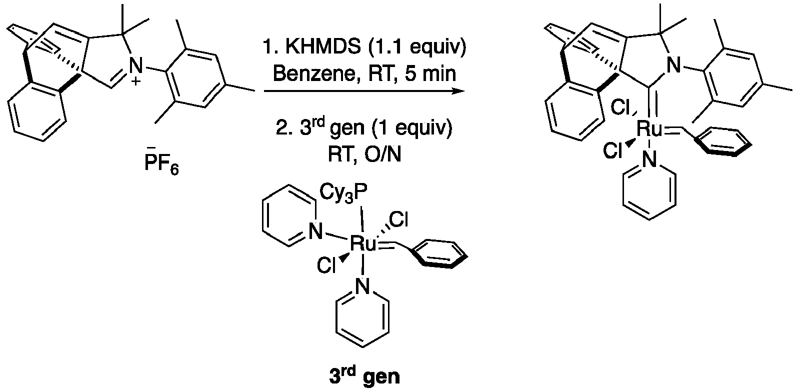

In a glove box, CABC [PF₆] (80.2 mg, 0.150 mmol, 1.1 equiv) was dissolved in dry and degassed Benzene (2 mL) in an oven-dried Schlenk probe with KHMDS (31.4 mg, 0.157 mmol, 1.2 equiv). The mixture was allowed to stir for 5 min at RT. The mixture was then filtered through a glass filter and Grubbs 3^{rd} gen complex (100 mg, 0.143 mmol, 1 equiv) (prepared according to previously reported procedure starting from Grubbs I and excess pyridine in toluene¹) was then added. The reaction was monitored by NMR (¹H and ³¹P) and was stirred overnight RT. The mixture was filtered through celite and then purified by precipitation in toluene/pentane followed by washing with Pentane to afford a dark green solid (67 mg, 64% yield).

¹H NMR (400 MHz, C₆D₆) δ 20.87 (s, 1H), 9.06 (d, *J* = 7.6 Hz, 2H), 8.49 (d, *J* = 5.5 Hz, 2H), 7.76 (d, *J* = 7.8 Hz, 2H), 7.22 (d, *J* = 7.2 Hz, 2H), 6.99 (s, 3H), 6.95 (t, *J* = 8.2 Hz, 2H), 6.87 (t, *J* = 7.3 Hz, 2H), 6.66 (t, *J* = 7.5 Hz, 2H), 6.56 (t, *J* = 7.7 Hz, 1H), 6.32 (d, *J* = 6.0 Hz, 1H), 6.24 (t, *J* = 6.7 Hz, 2H), 4.94 (d, *J* = 6.0 Hz, 1H), 2.67 (s, 6H), 2.13 (s, 3H), 1.03 (s, 7H).

### Example 18: Preparation of Synthesis of N-Mesityl CABC Grela type Ru complex (Ru-6) [JAT-87]

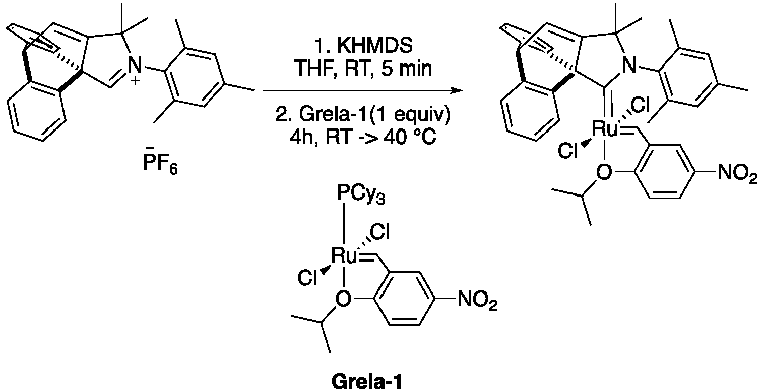

In a glove box, CABC [PF₆] (112.7mg, 0.21 mmol, 1.5 equiv) was dissolved in dry and degassed THF in an oven-dried Schlenk with KHMDS (42.8 mg, 0.22 mmol, 1.5 equiv). The mixture was allowed to stir for 5 min at RT. To this mixture, Grela-1 complex (99.7 mg, 0.15 mmol, 1 equiv) (prepared according to previously reported procedure starting from complex M1 (Dichloro(3-phenyl-1H-inden-1-ylidene)bis(tricyclohexylphosphine)ruthenium(II)) purchased from Umicore and 1-isopropoxy-4-nitro-2-vinylbenzene²) was then added. The mixture was stirred during 3 hours at RT then 1h at 40 °C. The reaction was monitored by NMR (¹H and ³¹P) and purified by column chromatography (pent/acetone 9:1). The isolate was then washed with hexane and precipitated in a hexane/DCM system to afford a dark green solid (13.3 mg, 13% yield).
¹H NMR (400 MHz, CDCl₃) δ 18.27 (s, 1H), 8.52 (dd, *J* = 9.2, 2.7 Hz, 1H), 8.13 (d, *J* = 2.7 Hz, 1H), 7.99 - 7.92 (m, 2H), 7.47 (dd, *J* = 7.2, 1.3 Hz, 2H), 7.19 (s, 2H), 7.14 (d, *J* = 9.2 Hz, 1H), 7.06 (td, *J* = 7.5, 1.1 Hz, 2H), 6.85 (td, *J* = 7.6, 1.3 Hz, 2H), 6.79 (d, *J=* 6.0 Hz, 1H), 5.37 - 5.30 (m, 1H), 5.24 (hept, *J* = 6.2 Hz, 1H), 2.46 (s, 6H), 2.43 (s, 3H), 1.58 (d, *J* = 6.1 Hz, 6H), 1.37 (s, 7H).

### Example 19: Synthesis of N-Mesityl CABC Z-stereoretentive complex (Ru-7) [JM-718]

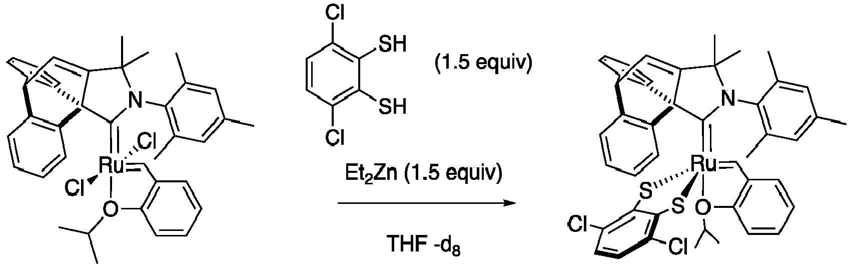

In a flame dried vial, 3,6-dichlorobenzene-1,2-dithiol (13.2 mg, 0.062 mmol, 1.5 equiv) and Et2Zn (0.91M in Hex, 61 µL, 0.056 mmol, 1.5 equiv) was added. The mixture was placed in the glove box. Dry and degassed THF (1.15mL) was added and the mixture was stirred for 5 min at RT. Afterwards, *N*-Mesityl CABC Ru-Hoveyda type complex (15.2mg, 0.021 mmol, 1 equiv), dissolved in THF-d8, was added to the previous mixture and the conversion was monitored by ¹H NMR. The crude solid was diluted in DCM and filtered through celite, washed with pentane and filtered through cotton. Volatiles were removed under vacuum. Pentane was added to help other solvent evaporation to afford brown solid with the characteristic signal at ¹H NMR (400 MHz, THF-d₈) δ 14.86 (s, 1H), that was used as a crude mixture in catalysis (see part 1.4.4).

### Example 20: Synthesis of N-Adamantyl CABC Hoveyda type Pyrrolide Ru complex (Ru-8) [JAT-215]

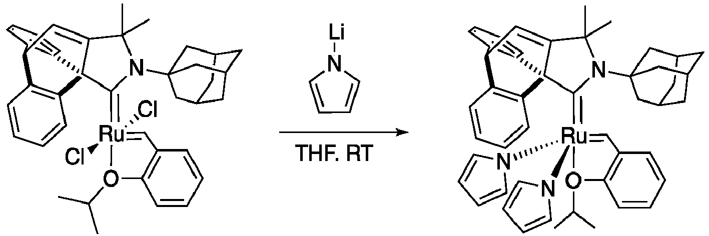

In glovebox, a 8 mL vial was charged with Ru-2 (66 mg, 0.10 mmol, 1 equiv) in THF (1 mL, solution A). Another vial was charged with lithium pyrrolide (26 mg, 0.34 mmol, 3.5 equiv) in THF (1.5 mL, solution B). Solution A was chilled in a glovebox freezer for 2-3 minutes. Solution A was then taken out of the freezer, and Solution B was transferred into solution A using a syringe over one minute. The resulting mixture was stirred for 16 hours at room temperature and monitored by NMR to ensure full conversion. Over a period of 2 minutes and with vigorous stirring, the reaction solution was added dropwise to a 60 mL vial pre-charged with pentane (40 mL), which led to the generation of a large amount of precipitate. The reaction vessel (the 8 mL vial) was rinsed with benzene (2 mL), and the resulting solution was transferred to the 60 mL vial as well. The resulting suspension was filtered through glass filter and then washed with pentane (5 mL). The solid was then recovered from the filter by THF (6 mL) rewashed with pentane and dried under vacuum to afford a brown solid (41 mg, 57 % yield).
¹H NMR (400 MHz, THF-d₈) δ 17.77 (s, 1H), 7.68 (ddd, *J* = 8.7, 7.1, 1.9 Hz, 1H), 7.45 (d, *J* = 8.4 Hz, 1H), 7.19 (dd, *J* = 7.2, 1.3 Hz, 2H), 6.88 (t, *J* = 7.3 Hz, 1H), 6.83 (dd, *J* = 7.6, 1.8 Hz, 1H), 6.70 (ddd, *J* = 8.5, 6.6, 1.6 Hz, 2H), 6.39 (t, *J* = 1.9 Hz, 4H), 6.23 (d, *J* = 7.7 Hz, 2H), 6.17 (td, *J* = 7.6, 1.4 Hz, 2H), 5.82 (t, *J* = 1.8 Hz, 4H), 5.38 (h, *J* = 6.1 Hz, 1H), 5.13 (d, *J* = 6.0 Hz, 1H), 2.90 (s, 5H), 2.33 (s, 3H), 1.88 (d, *J* = 12.2 Hz, 3H), 1.81 - 1.70 (m, 6H), 1.70 (s, 6H), 1.53 (d, *J* = 6.1 Hz, 6H).
¹³C NMR (101 MHz, THF-d₈) δ 303.4, 262., 159.1, 152.9, 145.6, 143.5, 140.5, 129.4, 127.8, 124.3, 123.2, 122.9, 121.9, 121.8, 120.9, 119.7, 112.0, 105.4, 73.7, 73.6, 73.0, 50.1, 33.7, 32.2, 30.3, 28.9, 20.4, 19.7, 11.5.

### Example 21: Synthesis of N-Mesityl CABC Blechert type Ru complex (Ru-9) [JAT-213]

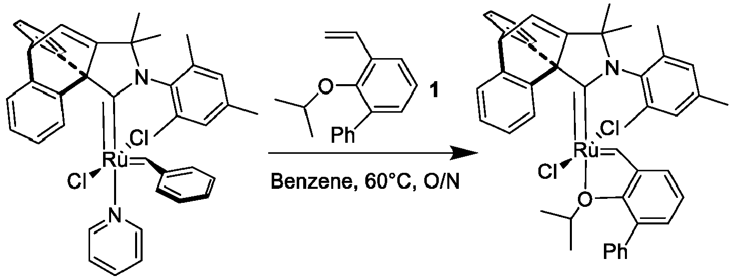

In a glove box, **Ru-5** complex (46 mg, 0.06 mmol, 1 equiv) was dissolved in dry and degassed Benzene in an oven-dried Schlenk, then Styrenyl **1** (18.1 mg, 0.08 mmol, 1.2 equiv) was added and allowed to stir overnight at 60°C. The reaction was monitored by NMR ¹H and ³¹P. The mixture was purified by column chromatography (eluent: toluene), then washed with Hexane and precipitated in Hexane/DCM to afford a green solid (13 mg, 26% yield).
¹H NMR (400 MHz, CDCl₃) δ 18.35 (s, 1H), 8.13 (dd, *J* = 7.7, 1.1 Hz, 2H), 7.54 - 7.47 (m, 2H), 7.46 - 7.36 (m, 5H), 7.23 (dd, *J* = 7.6, 1.7 Hz, 1H), 7.15 (s, 2H), 7.03 (td, *J* = 7.4, 1.1 Hz, 2H), 6.94 (t, *J* = 7.5 Hz, 1H), 6.89 (td, *J* = 7.6, 1.3 Hz, 2H), 6.78 (d, *J* = 6.0 Hz, 1H), 5.30 (d, *J* = 5.9 Hz, 1H), 4.75 (p, *J* = 6.2 Hz, 1H), 2.47 (s, 6H), 2.38 (d, *J* = 2.9 Hz, 3H), 1.37 (s, 6H), 1.16 (d, *J* = 6.2 Hz, 6H), 1.06 (d, *J* = 6.1 Hz, 1H).

### Thermal Stability of Ru-1

In a J. Young NMR tube, the Ru-1 complex (15.2 mg, 0.021 mmol, 1 equiv) was dissolved in dry and degassed Tol-d₈ (0.5 mL) and trimethoxybenzene (2 mg, 0.068 mmol, 3.3 equiv) as an internal standard were introduced in the reaction mixture, which was allowed to stir afterwards at 60°C for 4 hours, then at 80°C and finally at 105°C, until the start of the degradation was observed by NMR ¹H. After 8 days at 105°C, 96% of the initial complex was still present in the reaction mixture.

### CATALYTIC PERFORMANCE OF THE COMPLEXES

### Ring-opening metathesis polymerization of norbornene

In a flame-dried vial, norbornene (14.1 mg, 0.150 mmol, 1 equiv) with trimethoxybenzene as internal standard (8.3 mg, 0.049 mmol, 0.33 equiv) were charged. The desired [Ru] complex (5 mol%) was dissolved in DCE (1 mL) and the corresponding volume was introduced in the reaction mixture, which was allowed to stir afterwards at RT. The completion was monitored by NMR (¹H).

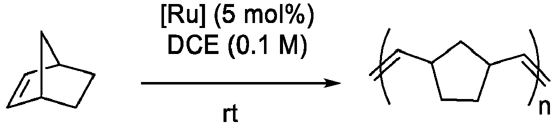

| | | Conversion (%) | |
|---|---|---|---|
| Catalyst | Ru-1 | Ru-3 | Ru-4 |
| Time | | | |
| 5 min | 11 | 18 | 84 |
| 15 min | 17 | 88 | |
| 30 min | 21 | | 99 |
| 45 min | 26 | 100 | |
| 1h | 28 | | 100 |
| 2h | 38 | | |
| 3h | 50 | | |

### Ring-closing metathesis of diethyl diallylmalonate

In a flame-dried vial, diethyl diallylmalonate (48 µL, 0.2 mmol, 1 equiv) was diluted in dry and degassed solvent of choice together with trimethoxybenzene as internal standard (11.1 mg, 0.061 mmol, 0.33 equiv). The [Ru] complex (X mol%) was dissolved in the same solvent (1 mL) and the requested volume introduced in the reaction mixture, which was allowed to stir afterwards at the indicated temperature. The completion was monitored by NMR (¹H).

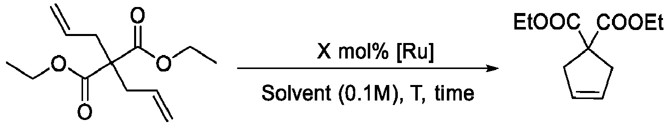

| | | | Conversion (% ) | | |
|---|---|---|---|---|---|
| Catalyst/Loading | Ru-1 (1 mol%) | Ru-3 (5 mol%) | Ru-4 (5 mol%) | Ru-5 (3 mol%) | Ru-6 (5 mol%) |
| Temperature(°C)/Solvent | 110/Tol | 40/DCE | 40/DCE | 110/Tol | 75/DCE |
| Time | | | | | |
| 1h | 28 | 5 | 30 | 22 | 1 |
| 2h | 36 | 5 | 64 | | |
| 3h | 44 | 24 | 81 | | |
| 18h | 76 | 42 | 100 | 26 | 93 |

### SM of 1-dodecene

In a flame-dried vial, 1-dodecene (40 µL, 0.225 mmol, 1 equiv) was diluted in dry and degassed solvent (1 mL) with trimethoxybenzene as an internal standard (0.33 equiv) and [Ru] complex solution (X mol%). The reaction mixture was allowed to stir at the indicated temperature and time. The reaction completion was monitored by NMR ¹H.

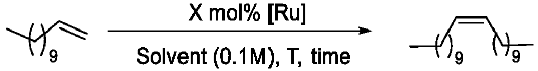

| | Conversion (%) | | | |
|---|---|---|---|---|
| Catalyst/Loading | Ru-1 (1 mol%) | Ru-1 (1 mol%) | Ru-3 (5 mol%) | Ru-4 (5 mol%) |
| Temperature(°C)/Solvent | 50/DCE | 110/Tol | 40/DCE | 40/DCE |
| Time | | | | |
| 1h | 3 | | 7 | 22 |
| 2h | 9 | 27 | 11 | 29 |
| 3h | 10 | | 21 | 36 |
| 18h | 43 | | 30 | 56 |
| 24h | | 70 | 30 | 60 |

### Ring-opening cross metathesis with Ru-7

In the glovebox, a flame dried Schlenk charged with 5-Norbornene-2,3-dimethanol (32.6 mg, 0.211 mmol, 1 equiv) and distilled and degassed styrene (0.42mL, 3.666 mmol, 17 equiv) were charged together with toluene (2 mL) and a Ru-7 solution (0.30 mL, 0.0099 mmol, 4.7 mol%) and the mixture was stirred overnight at 70 °C. The conversion and E/Z ratio were monitored by GC/MS to afford the desired product with 1/99 E/Z ratio at 40 % conversion of the starting material.

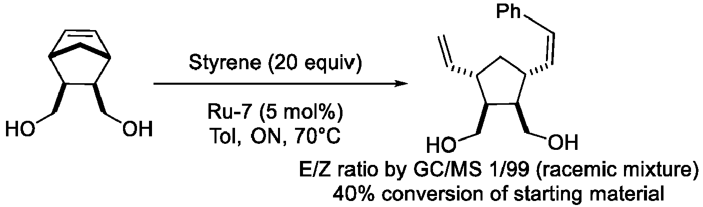

## Claims

1. An iminium salt having the following formula (I): 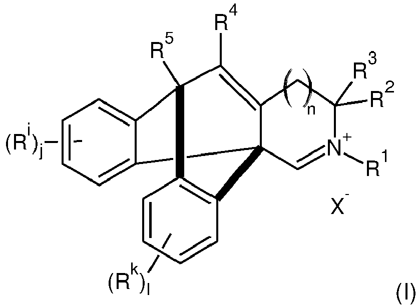 wherein:
- n = 0 or 1;
- R¹ is a (C₆-C₁₄)aryl group, a (C₁-C₆)alkyl group or a (C₈-C₂₀)cycloalkyl group, said aryl group being optionally substituted with at least one substituent chosen from the group consisting of: halogen, (C₁-C₆)alkoxy group, (C₆-C₁₀)aryl group, and (C₁-C₆)alkyl group, said alkyl group being optionally substituted with one or several phenyl group(s) or optionally interrupted by at least one oxygen, nitrogen or sulfur atom, or said alkyl group being optionally substituted with at least one substituent chosen from the group consisting of: (C₆-C₁₆)aryl group, (C₂-C₆)alkenyl group, (C₂-C₆)alkynyl group, hydroxyl, (C₆-C₁₀)arylcarbonyl, (C₁-C₆)alkoxycarbonyl, -C=C-Si((C₁-C₆)alkyl)₃, and -O-Si((C₁-C₆)alkyl)₃;
or R¹ is a -NR'ₐR'_{b} group, R'ₐ and R'_{b} being independently from each other selected from the group consisting of: H, (C₁-C₆)alkyl, and (C₆-C₁₀)aryl, or R'ₐ and R'_{b} form together with the nitrogen atom carrying them a N(CH₂)₂₊ₘ heterocyclyl ring, m being 0 or an integer comprised from 1 to 6;
- R² is H, a (C₆-C₁₀)aryl group or a (C₁-C₆)alkyl group;
- R³ is a (C₁-C₆)alkyl group; or R² and R³ may together form, with the carbon atom carrying them, a (C₃-C₆)cycloalkyl;
- R⁴ is selected from the following groups: H, (C₆-C₁₀)aryl, and (C₁-C₆)alkyl,
- R⁵ is selected from the following groups: H, (C₆-C₁₀)aryl, and (C₁-C₆)alkyl,
- j is 0 or an integer comprised from 1 to 4;
- i is an integer varying from 1 to j, and each Rⁱ, identical or different, is selected from the following groups: H, (C₆-C₁₀)aryl, and (C₁-C₆)alkyl,
- l is 0 or an integer comprised from 1 to 4;
- k is an integer varying from 1 to l, and each R^{k}, identical or different, is selected from the following groups: H, (C₆-C₁₀)aryl, and (C₁-C₆)alkyl; and
- X⁻ is a counteranion.

2. The iminium salt of claim 1, wherein j=l=0.

3. The iminium salt of claim 1 or 2, wherein R⁴ is H.

4. The iminium salt of any one of claims 1 to 3, wherein R² and R³ are (C₁-C₆)alkyl groups, and are preferably identical.

5. The iminium salt of any one of claims 1 to 4, wherein R¹ is a (C₆-C₁₄)aryl group, preferably a phenyl group, a (C₁-C₆)alkyl group or a (C₈-C₂₀)cycloalkyl group, said aryl group being optionally substituted with at least one substituent chosen from the group consisting of: halogen, (C₆-C₁₀)aryl group, and (C₁-C₆)alkyl group.

6. The iminium salt of any one of claims 1 to 5, having one of the following formulae:

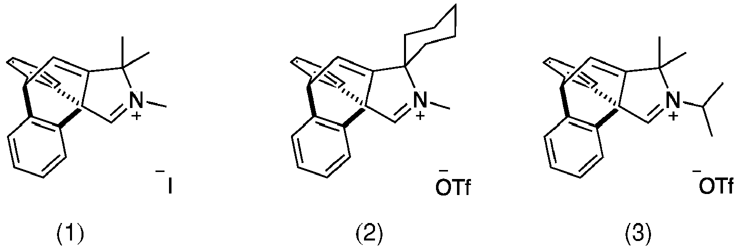

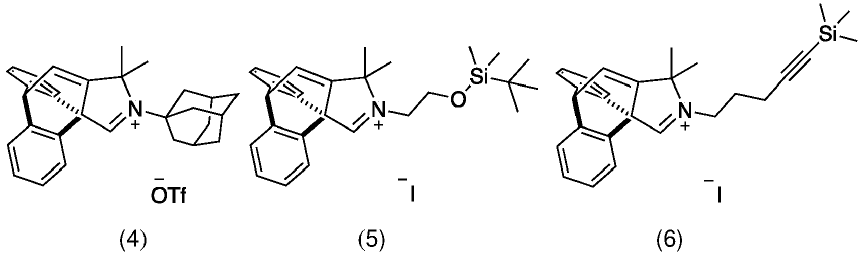

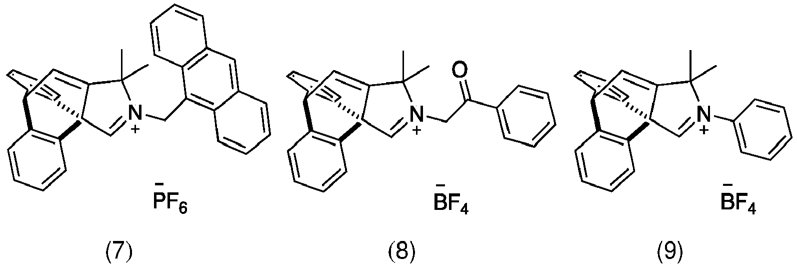

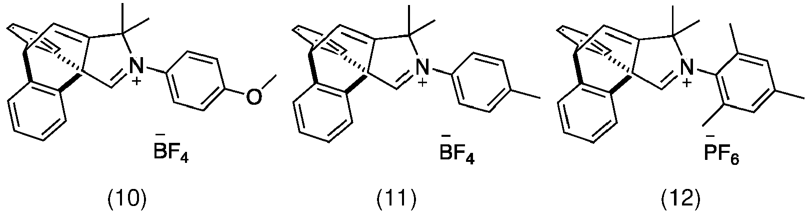

7. A ruthenium complex having the following formula (II): 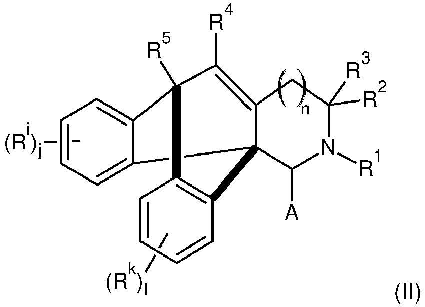 wherein:
- n, j, k, Rⁱ, R^{k}, R¹, R², R³, R⁴ and R⁵ are as defined in formula (I) in any one of claims 1 to 6, and
- A is either a group of formula (1) or a group of formula (2): 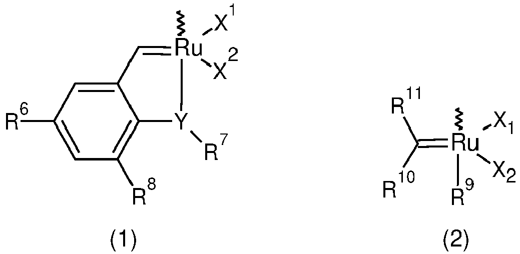
wherein:
- X¹ is an halogen atom, a (C₁-C₆)alkoxy group, a heteroaryl group or a -S-(C₆-C₁₀)aryl group;
- X² is an halogen atom, a heteroaryl group or a (C₁-C₆)alkoxy group;
or X¹ and X² may form together with the ruthenium atom carrying them a heterocycloalkyl group fused with a phenyl group, said phenyl group being possibly substituted with at least one halogen atom;
- Y is an oxygen or a sulfur;
- R⁶ is H or is selected from the following groups: nitro, cyano, (C₁-C₆)alkyl, cycloalkyl, (C₁-C₆)alkoxy, cycloalkyloxy, (C₆-C₁₀)aryl, heteroaryl, (C₆-C₁₀)aryloxy, heteroaryloxy, (C₁-C₆)alkylcarbonyl, arylcarbonyl, (C₁-C₆)alkoxycarbonyl, aryloxycarbonyl, carboxyl, amido, (C₁-C₆)alkylsulfonyl, arylsulfonyl, (C₁-C₆)alkylsulfinyl, arylsulfinyl, (C₁-C₆)alkylthio, arylthio, sulfonamide, halogen, -NRₐR_{b}, -SO₂-NRR', and -N(R_{c})-C(=O)-R_{c},
Rₐ and R_{b} being independently selected from H and (C₁-C₆)alkyl,
R and R' being selected from the following groups: (C₁-C₆)alkyl, (C₆-C₁₀)aryl, heteroaryl, and halo(C₁-C₆)alkyl,
R_{c} being H or being selected from the following groups: (C₁-C₆)alkyl, (C₆-C₁₀)aryl, and heteroaryl,
R_{d} being H or selected from the following groups: (C₁-C₆)alkyl, halo(C₁-C₆)alkyl, cycloalkyl, (C₆-C₁₀)aryl, heteroaryl, (C₁-C₆)alkoxy, cycloalkyloxy, (C₆-C₁₀)aryloxy, and heteroaryloxy;
- R⁷ is a (C₁-C₆)alkyl group or a (C₆-C₁₀)aryl group;
- R⁸ is H, a (C₆-C₁₀)aryl group or a (C₁-C₆)alkyl group;
- R⁹ is a heteroaryl group, or a group -P(R¹²)₃ or P(OR¹²)₃, R¹² being chosen from the group consisting of: (C₁-C₆)alkyl, (C₆-C₁₀)aryl, and cycloalkyl;
- R¹⁰ is a (C₆-C₁₄)aryl group, preferably a phenyl group, said aryl group being optionally substituted with at least one substituent chosen from the group consisting of: halogen, (C₆-C₁₀)aryl group, and (C₁-C₆)alkyl group;
- R¹¹ is H or a (C₆-C₁₄)aryl group, or R¹⁰ and R¹¹ may together form a indenyl group optionally substituted with at least one substituent chosen from the group consisting of: (C₆-C₁₀)aryl group, and (C₁-C₆)alkyl group;

8. The ruthenium complex of claim 7, having the following formula (III): 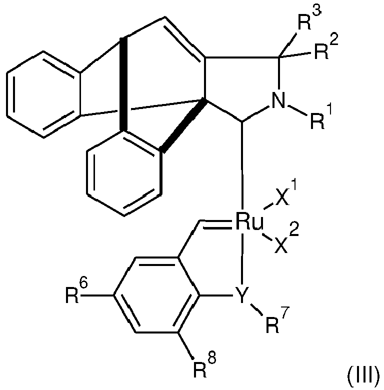 wherein:
- X¹ is an halogen atom or a (C₁-C₆)alkoxy group;
- X² is an halogen atom or a (C₁-C₆)alkoxy group;
- Y is an oxygen or a sulfur, preferably an oxygen;
- R¹ is a (C₆-C₁₀)aryl group, a (C₈-C₂₀)cycloalkyl group or a (C₁-C₆)alkyl group, said aryl group being optionally substituted with at least one substituent chosen from the group consisting of (C₁-C₆)alkyl;
- R² is a (C₆-C₁₀)aryl group or a (C₁-C₆)alkyl group;
- R³ is a (C₁-C₆)alkyl group;
- R⁶ is H, nitro or a (C₁-C₆)alkyl group.
- R⁷ is a (C₁-C₆)alkyl group, such as isopropyl; and
- R⁸ is H, a (C₆-C₁₀)aryl group or a (C₁-C₆)alkyl group.

9. The ruthenium complex of claim 7 or 8, wherein X₁ and X₂ are halogen atoms, preferably Cl.

10. The ruthenium complex of any one of claims 7 to 9, wherein R¹ is a phenyl group, optionally substituted with at least one substituent chosen from the group consisting of (C₁-C₆)alkyl, preferably a phenyl group substituted with three alkyl groups, such as methyl groups, or R¹ is a (C₈-C₂₀)cycloalkyl group or a (C₁-C₆)alkyl group.

11. The ruthenium complex of any one of claims 7 to 10, wherein R² is a (C₁-C₆)alkyl group.

12. The ruthenium complex of any one of claims 7 to 11, wherein R² and R³ are identical, and are preferably a methyl group.

13. The ruthenium complex of any one of claims 7 to 12, wherein R⁶ is H or nitro.

14. The complex of any one of claims 7 to 13, having one of the following formulae:

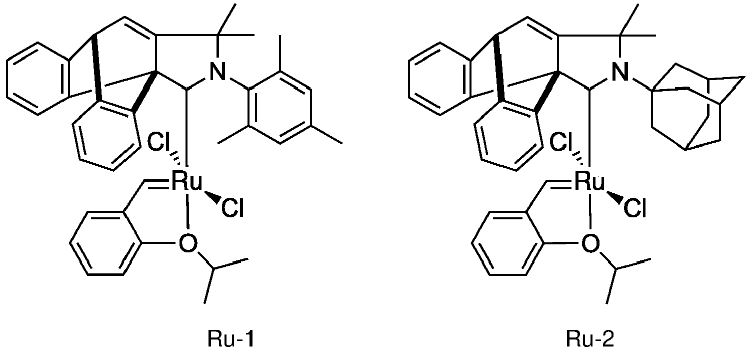

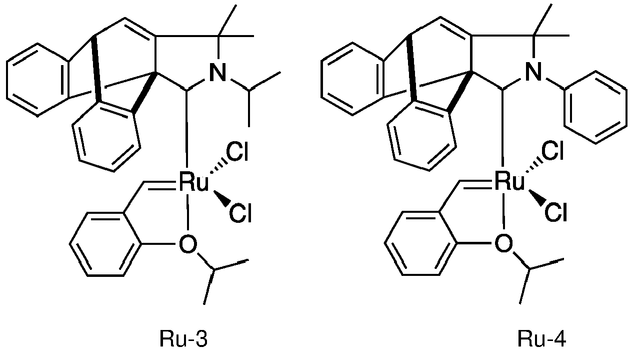

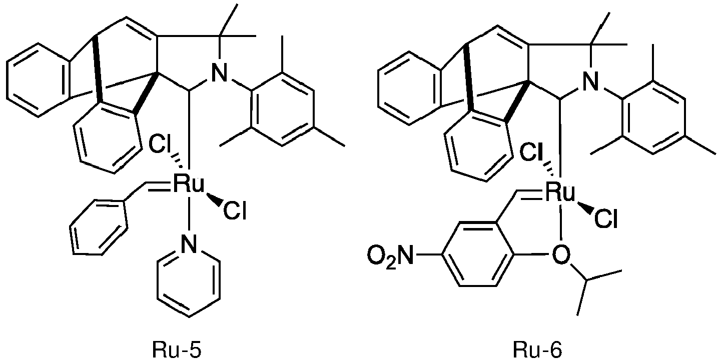

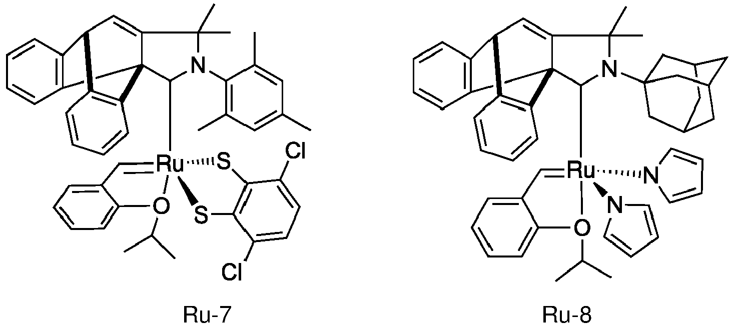

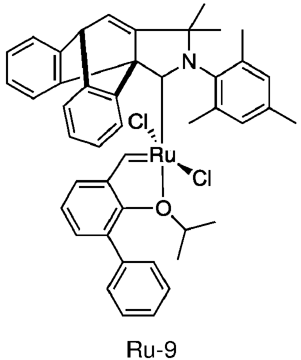

15. The use of the complex of any one of claims 7 to 14 as a catalyst, preferably as a catalyst in olefin metathesis.
